(19) Europäisches Patentamt
European Patent Office
Office européen des brevets

(11) Veröffentlichungsnummer: **0 019 739**
**B1**

# (12) EUROPÄISCHE PATENTSCHRIFT

(45) Veröffentlichungstag der Patentschrift: **18.07.84**

(51) Int. Cl.³: **C 07 D 213/65**, A 61 K 31/44

(21) Anmeldenummer: **80102347.4**

(22) Anmeldetag: **30.04.80**

## (54) 2-Aminoalkyl-5-pyridinole, Verfahren zu ihrer Herstellung und pharmazeutische Präparate die diese Verbindungen enthalten.

(30) Priorität: **03.05.79 US 35668**

(43) Veröffentlichungstag der Anmeldung: **10.12.80 Patentblatt 80/25**

(45) Bekanntmachung des Hinweises auf die Patenterteilung: **18.07.84 Patentblatt 84/29**

(84) Benannte Vertragsstaaten: **AT BE CH DE FR IT LI LU NL SE**

(56) Entgegenhaltungen: **US - A - 3 952 101**

(73) Patentinhaber: **CIBA-GEIGY AG, Postfach, CH-4002 Basel (CH)**

(72) Erfinder: **Mizzoni, Renat Herbert, Dr., 1001 Forrest Highlands Roads, Prescott Arizona 86301 (US)**

(74) Vertreter: **Zumstein, Fritz sen., Dr. et al, Bräuhausstrasse 4, D-8000 München 2 (DE)**

EP 0 019 739 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Gemäß dem US-Patent 3 952 101 haben die sekundären 2-Amino-1-hydroxyäthyl-5-pyridinole oder »α-Aminomethyl-5-hydroxy-2-pyridinmethanole eine direkte Bronchodilatator-Wirkung mit minimaler Herzstimulation«, d. h. »sie weisen eine stärkere Wirkung auf die glatte Muskulatur der Atmungsorgane als auf den Herzmuskel auf«.

Es ist überraschend gefunden worden, daß bei Weglassung der genannten aliphatischen 1-Hydroxy- oder der methanolischen Gruppe, die Wirkung ins Gegenteil umschlägt. Es werden nämlich die herzwirksamen Mittel der vorliegenden Erfindung erhalten, welche eine vernachlässigbare Bronchodilatator-Wirkung aufweisen.

Die vorliegende Erfindung betrifft daher neue sekundäre 2-Aminoalkyl-5-pyridinole der allgemeinen Formel I

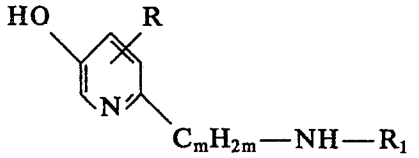

(I)

worin R Wasserstoff oder Alkyl mit 1—4 Kohlenstoffatomen, vorzugsweise Methyl, bedeutet, m für eine ganze Zahl von 2 bis 4 steht, und $R_1$ Alkyl, Cycloalkyl oder Alkenyl mit jeweils höchstens 7 Kohlenstoffatomen bedeutet, wobei sich in den Alkenylresten die Doppelbindung in 2- oder 3-Stellung befindet, ihre Säureadditionssalze, insbesondere therapeutisch verwendbare Säureadditionssalze, Verfahren zu ihrer Herstellung, pharmazeutische Präparate die diese Verbindungen enthalten, sowie die Verbindungen zur therapeutischen Verwendung.

Bedeutet R Alkyl mit 1—4 C-Atomen, vorzugsweise Methyl, so kann diese Gruppe in irgendeiner der freien Stellungen 2-, 4- oder 6- des Pyridinrestes stehen. Der Alkylrest R ist z. B. Methyl, Äthyl, n- oder Isopropyl, n- oder Isobutyl. Das Symbol R bedeutet jedoch in erster Linie Wasserstoff.

Die Alkylgruppe $C_mH_{2m}$ bedeutet vorzugsweise 1,2-Propylen, aber auch Äthylen, 1,3-Propylen, 1,2-, 1,3- oder 1,4-Butylen.

Die Gruppe $R_1$ weist jeweils jöchstens 7 C-Atome auf und steht vorzugsweise für Alkyl, z. B. Methyl, Äthyl, n- oder i-Propyl, n-, iso- oder tert.-(Butyl, Pentyl, Hexyl oder Heptyl); insbesondere für Isopropyl.

Die Gruppe $R_1$ bedeutet weiter Alkenyl, z. B. Allyl, Methallyl, 2- oder 3-(Butenyl, Pentenyl, Hexenyl oder Heptenyl), oder Cycloalkyl, z. B. Cyclopropyl, Cyclobutyl, Cyclopentyl, Cyclohexyl oder Cycloheptyl; insbesondere Cyclopropyl oder Cyclohexyl.

Die organischen Reste $R_1$ sind solche mit höchstens 7, vorzugsweise 4, insbesondere 1 oder 2 Kohlenstoffatomen.

Die Säureadditionssalze der dibasischen Verbindungen der Formel I sind vorzugsweise therapeutisch verwendbare Säureadditionssalze, z. B. solche der weiter unten genannten Säuren.

Die Verbindungen der Erfindung zeigen wertvolle pharmakologische Wirkungen, z. B. antihypertensive, aber insbesondere kardioprotektive, z. B. antiischämische (d. h. antianginale) Eigenschaften.

Diese können in Tierversuchen, vorzugsweise an Säugetieren, z. B. Ratten, Katzen oder Hunden, oder ihren isolierten Organen nachgewiesen werden. Die neuen Verbindungen können ihnen enteral oder parenteral, vorzugsweise oral oder intravenös, z. B. durch Gelatine-Kapseln oder in Form von Stärke enthaltenden Suspensionen bzw. wäßrigen Lösungen, verabreicht werden. Die verwendete Dosis kann in einem Bereich zwischen 1 und 200 mg/kg/Tag, vorzugsweise 3 und 30 mg/kg/Tag bei intravenösen oder zwischen 10 und 100 mg/kg/Tag bei peroraler Verabreichung, liegen.

Schwache antihypertensive Effekte können an spontan hypertensiven Ratten, oder an renal hypertensiven Hunden beobachtet werden. Diese Wirkungen können entweder durch Sphygmomanometrie am Rattenschwanz, oder direkt mit einem Katheter, der in die femurale Arterie eines Hundes eingeführt ist, und durch ein Übertragungsinstrument den Blutdruck in mm Hg angibt, registriert werden.

Die kardioprotektive Wirkung der neuen Verbindungen ist ähnlich wie diejenige des Nitroglycerins, Propanolols und/oder Verapamils. Diese Mittel unterdrücken die elektrokardiographische (EKG) Manifestation (ST-T-Erhöhung) der myokardialen Ischämie, welche durch den vorübergehenden Koronarverschluß an narkotisierten Katzen oder wachen Hunden, hervorgerufen wird. Es wird an den Hunden vorher eine linksseitige Eröffnung der Brusthöhle unter Pentobarbital-Narkose und künstlicher Beatmung durchgeführt. Der Herzbeutel wird geöffnet und ein Segment der linken Koronararterie freigelegt. Dies ermöglicht die Einpflanzung eines aufblasbaren Ballon-Verschlusses aus Siliciumkautschuk um das freigelegte Arteriensegment. Das Ableitungsrohr des Verschlusses wird durch den Hinterteil des Halses an das Schulterblatt ninausgeführt und ein Schutzmantel angebracht. Man läßt die Hunde in 7 bis 10 Tagen genesen, wobei sie während der ersten vier Tage nach der Operation Antibiotika erhalten. Es wird dann bei den Hunden eine Kontrolle des Arterienverschlusses vorgenommen, wobei jedoch keine EKG-Aufnahmen stattfinden. Im Test wird die Arterie in Intervallen von 5 Minuten zwei- bis dreimal für je 1—1,5 Minuten verschlossen und die Veränderung im ST-T-Segment der EKG-Leitung II registriert. Man berechnet die Durchschnittswerte vor der Verabreichung des Wirkstoffes und,

in bestimmten Intervallen, nach der Wirkstoffbehandlung. Die Ergebnisse werden als Verhältnis von Werten, welche bei behandelten und nicht behandelten (Kontroll-)-Tieren erhalten werden, ausgedrückt. Auch ein permanenter Koronarverschluß wird durchgeführt. Es werden dann venöse Blutproben nach 3, 6 und 24 Stunden nach dem Arterienverschluß für die Bestimmung von CPK-Werten (Kreatin-Phospho-Kinase) abgenommen. Die Hunde werden danach narkotisiert, mit Trypanblau-Farbstoff injiziert, mit einer Überdosis an Pentobarbital-natrium eingeschläfert und ihre Herzen für die Beurteilung vom nekrotischen Gewebe seziert. Gemäß den erhaltenen Resultaten rufen die Verbindungen der Erfindung eine signifikante Verminderung der elektrischen, enzymatischen und morphologischen Veränderungen (Größe des Infarkts) hervor, welche durch Koronarverschluß an wachen Hunden ausgelöst worden sind. Die Verfahrensprodukte sind daher wertvolle kardioprotektive, insbesondere gegen Angina pectoris wirkende Mittel. Die neuen Verbindungen können überdies als Zwischenprodukte zur Herstellung von anderen wertvollen, insbesondere von pharmakologisch wirksamen Präparaten eingesetzt werden.

Bevorzugte Verbindungen sind diejenigen der Formel I, worin R Wasserstoff oder Methyl bedeutet, m für die Zahl 2 oder 3 steht, und $R_1$ Alkyl mit 2 bis 6 C-Atomen oder Cycloalkyl oder Alkenyl mit jeweils 3 bis 6 C-Atomen bedeutet, wobei sich in den Alkenylresten die Doppelbindung in 2- oder 3-Stellung befindet, und ihre Säureadditionssalze, insbesondere therapeutisch verwendbaren Säureadditionssalzen.

Besonders hervorzuheben sind Verbindungen der allgemeinen Formel II

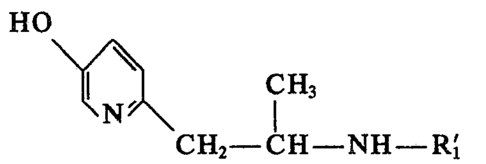

(II)

worin $R_1'$ Alkyl, Cycloalkyl oder Alkenyl mit jeweils 3 bis 6 C-Atomen bedeutet, wobei sich in den Alkenylresten die Doppelbindung in 2- oder 3-Stellung befindet, und ihre Säureadditionssalze, insbesondere therapeutisch verwendbaren Säureadditionssalze.

Bevorzugt sind weiter Verbindungen der allgemeinen Formel II, worin $R_1'$ i-propyl, tert.-Butyl, Allyl oder Cyclopropyl bedeutet, und ihre Säureadditionssalze, insbesondere pharmazeutisch verwendbaren Säureadditionssalze.

Die Verbindungen der vorliegenden Erfindung werden nach an sich bekannten Methoden, z. B. dadurch hergestellt, daß man

a) an eine Verbindung der allgemeinen Formel III

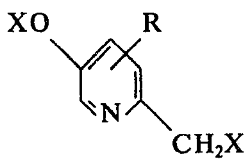

(III)

worin X ein Alkalimetallatom oder eine Erdalkalimetall-Halogengruppe bedeutet, eine Verbindung der allgemeinen Formel IV

$$C_{m-1}H_{2m-2}{=}N-R_1 \qquad (IV)$$

addiert und das Produkt der allgemeinen Formel I aus dem erhaltenen Metallsalz freisetzt, oder

b) in einer Verbindung der allgemeinen Formel V

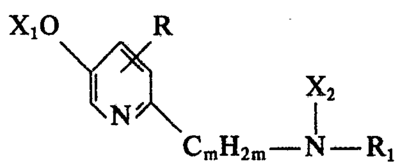

(V)

worin jedes der Symbole $X_1$ und $X_2$ Wasserstoff oder das Acylradikal einer aliphatischen oder aromatischen Carbonsäure oder Sulfonsäure bedeutet, mit der Maßgabe, daß mindestens eines dieser Symbole für ein Acylradikal steht, das Acylradikal $X_1$ und/oder $X_2$ durch Solvolyse oder Hydrogenolyse

durch Wasserstoff ersetzt, oder

c) eine Schiff'sche Base der allgemeinen Formel VI oder VIa

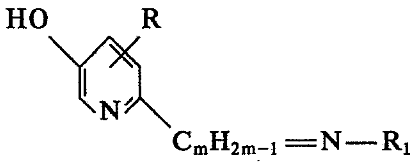

(VI)

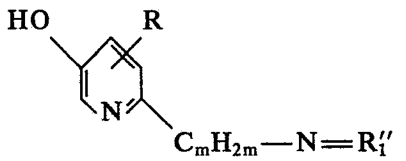

(VIa)

wobei das mit dem Stickstoffatom verbundene Kohlenstoffatom des Restes $R_1''$ ein Wasserstoffatom weniger als $R_1$ aufweist, reduziert, oder

d) ein primäres Amin der allgemeinen Formel VII

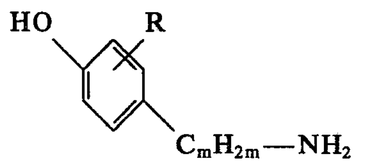

(VII)

mit einem reaktionsfähigen Ester des Alkohols $R_1-OH$ in Gegenwart einer starken Base kondensiert, oder

e) ein einem Amin der allgemeinen Formel I entsprechendes Amid, worin ein der sekundären Aminogruppe benachbartes Kohlenstoffatom anstelle von zwei Wasserstoffatomen ein Sauerstoffatom aufweist, reduziert und, wenn erwünscht, eine erhaltene Verbindung in eine andere Verbindung der Erfindung umwandelt, und/oder, wenn erwünscht, eine erhaltene Base in ein Säureadditionssalz oder ein erhaltenes Salz in die freie Base oder in ein anderes Säureadditionssalz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

Die genannten metallischen Substituenten X in einem Ausgangsstoff der allgemeinen Formel III sind vorzugsweise Lithium, Natrium oder Halogenmagnesium. In diesem Ausgangsstoff ist R vorzugsweise Methyl, in erster Linie Wasserstoff.

Die Additionsreaktion des Verfahrens a) wird unter wasserfreien Bedingungen, vorzugsweise in polaren Lösungsmitteln, z. B. in offenkettigen oder cyclischen Äthern, wie Diäthyläther oder Tetrahydrofuran, und bei Temperaturen unter Zimmertemperatur, z. B. zwischen ungefähr 10 und —20°, durchgeführt.

Die Umwandlung eines erhaltenen Metallsalzes der allgemeinen Formel Ia

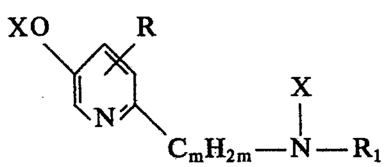

(Ia)

worin alle Symbole die oben angegebenen Bedeutungen haben, in die freie Verbindung der Formel I wird vorzugsweise mit Wasser oder mit verdünnten anorganischen oder organischen Säuren, z. B. mit wäßrigen Säuren, die mit den weiter unten aufgezählten, vorzugsweise bei Zimmertemperatur oder darunter, z. B. bei 0°, vorgenommen.

In einem Ausgangsstoff der Formel V, worin die Symbole $X_1$ und/oder $X_2$ Acylradikale bedeuten, sind diese vorzugsweise Niederalkanoyl oder Niederalkansulfonyl, unsubstituiertes oder niederalkyliertes niederalkoxyliertes und/oder halogeniertes Benzoyl, Phenylniederalkanoyl, Benzolsulfonyl oder Carbobenzyloxy, z. B. Acetyl, Propionyl, Phenyl-acetyl, Methansulfonyl, Benzoyl, p-Toluyl, p-Anisoyl, m-Chlorbenzoyl, Benzolsulfonyl, Toluolsulfonyl oder Carbobenzyloxy (Benzyloxyformyl). Hier werden somit unter »Acyl« die Acylradikale von aliphatischen oder aromatischen Carbonsäuren und Sulfonsäuren verstanden.

Der Ausdruck »nieder« definiert in den organischen Resten solche mit höchstens 7 vorzugsweise 4, insbesondere 1 oder 2 Kohlenstoffatomen.

Der Ersatz einer Acylgruppe $X_1$ und/oder $X_2$ durch Wasserstoff kann durch Behandlung mit solvolysierenden oder hydrogenolysierenden Mitteln durchgeführt werden. Die Solvolyse umfaßt z. B. die Hydrolyse, Alkoholyse oder Ammonolyse. Die Hydrolyse der genannten Ausgangsstoffe der Formel V wird vorzugsweise bei erhöhten Temperaturen, z. B. bei 40 bis 120°, durchgeführt. Man arbeitet in wäßrigen Medien, vorzugsweise mit verdünnten anorganischen oder organischen Säuren bzw. Basen, z. B. wäßrigen Alkalien oder Säuren, z. B. in den weiter unten genannten. Die Alkoholyse wird vorzugsweise mit Niederalkanolen, z. B. Methanol oder Äthanol, in Gegenwart von starken anorganischen Basen, z. B. wäßrigen Alkalimetallhydroxyden oder -carbonaten, oder von anorganischen Säuren, z. B. Halogenwasserstoffsäuren, wie Chlorwasserstoff-, Bromwasserstoff- oder Schwefelsäure, vorgenommen. Die Ammonolyse wird z. B. durch Behandlung mit wäßrigem Ammoniak durchgeführt. In den genannten Carbobenzyloxy-Verbindungen der Formel V kann die Carbobenzoxygruppe auch hydrogenolytisch durch Wasserstoff ersetzt werden. Man arbeitet gemäß den aus der Peptidsynthese bekannten Methoden, z. B. mit Wasserstoff in Gegenwart von Edelmetallkatalysatoren, z. B. Palladium- oder Platin-Katalysatoren.

Im Verfahren c) werden die Schiff'schen Basen der allgemeinen Formel VI oder VIa nach konventionellen Methoden, entweder mit katalytisch aktiviertem oder nascierendem Wasserstoff, wie Wasserstoff in Gegenwart von Palladium-, Platin- oder Nickel-Katalysatoren, oder mit elektrolytisch erzeugtem Wasserstoff, reduziert. Die Reduktion kann aber auch mit einfachen oder komplexen Leichtmetallhydriden, z. B. Boranen, Alan oder Alkalimetallborhydriden oder Alkalimetallcyanborhydriden, wie Natriumborhydrid oder Natriumcyanborhydrid, durchgeführt werden.

Die als Ausgangsstoffe verwendeten primären Amine der allgemeinen Formel VII werden vorzugsweise mit den genannten reaktionsfähigen Estern, welche von starken anorganischen Säuren, z. B. Halogenwasserstoffsäuren, wie Chlorwasserstoff-, Bromwasserstoff-, in erster Linie Jodwasserstoffsäure oder von einer oben genannten organischen Sulfonsäure abgeleitet sind, kondensiert. Die verwendeten, als Kondensationsmittel genannten starken Basen sind vorzugsweise tertiäre Amine, wie Tri-niederalkylamine, z. B. Di-isopropyl-äthylamin, oder cyclische Stickstoffbasen, z. B. Pyridin oder Lutidin. Man muß darauf achten, daß die gleichzeitige Quaternisierung der erhaltenen sekundären Amine der Formel I vermieden wird. Dies kann man z. B. erreichen, indem man keinen zu großen Überschuß an reaktionsfähigen Estern und/oder keine zu hohen Temperaturen (über Zimmertemperatur) verwendet.

Die Reduktion von Amid-Ausgangsstoffen des Verfahrens e) kann in an sich bekannter Weise mit stärkeren, für die Reduktion von Schiff'schen Basen VI genannten Leichtmetallhydriden, vorzugsweise mit Alan, in genannten starken Basen, oder mit Alkalimetallaluminiumhydriden, z. B. Lithiumaluminiumhydrid, Lithium- oder Natrium-tri-niederalkoxy-aluminiumhydrid oder Lithium- oder Natrium-bisalkoxy-alkoxy-aluminiumhydriden, z. B. Lithium-tri-tert.-butoxy-aluminiumhydrid oder Natrium-bis-(2-methoxyäthoxy)-aluminiumhydrid, durchgeführt werden.

Die Ausgangsstoffe sind bekannt, oder wenn neu, können sie nach an sich bekannten Methoden, z. B. wie in den Beispielen illustriert, hergestellt werden.

Die Ausgangsstoffe der Formel III können z. B. durch Umsetzung entsprechender 2-Methyl-5-pyridinole mit einer Niederalkyl-alkalimetallverbindung, z. B. mit n-Butyllithium, hergestellt werden. Die Verbindungen der Formel IV können z. B. durch Kondensation von niederen Alkanalen mit entsprechenden primären Aminen, erhalten werden.

Die genannten Acylderivate der Formel V können durch Kondensation einer Verbindung der Formel

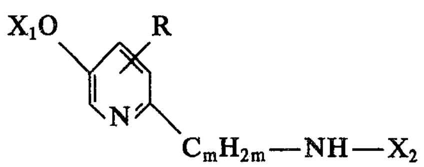

worin $X_1$ und $X_2$ die genannten Acylradikale von aliphatischen oder aromatischen Carbon- oder Sulfonsäuren bedeuten, mit einem oben genannten reaktionsfähigen Ester des Alkohols $R_1-OH$, hergestellt werden. Falls nötig, kann eine Vorstufe der obigen Verbindung, worin $X_1$ ein genanntes Acylradikal bedeutet und $X_2$ für Wasserstoff steht, z. B. mit Niederalkanoyl-, Niederalkansulfonyl-, Benzoyl-, Benzolsulfonyl- oder Benzyloxycarbonyl-halogeniden acyliert werden.

Die als Ausgangsstoffe verwendeten Schiff'schen Basen der allgemeinen Formel VI oder VIa können zweckmäßig durch Kondensation von Verbindungen der allgemeinen Formeln

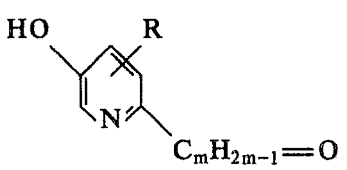

und $H_2N-R_1$

oder

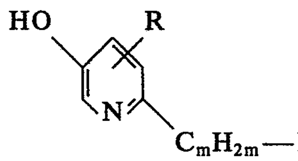 und $O{=}R_1''$

erhalten werden. Die Aldehyd- bzw. Keton-Verbindungen, welche als Vorstufen verwendet werden, können ähnlich wie die Verbindungen der Formel III, d. h. durch Kondensation der genannten Metallsalze des 5-Hydroxypikolins mit entsprechenden Alkancarbonsäureamiden oder -nitrilen, z. B. Formamid, Dimethylacetamid oder Propionitril, und Hydrolyse des Kondensats mit Wasser oder verdünnten Säuren, oder Hydrierung zu den genannten Aminen der allgemeinen Formel VII, hergestellt werden. Die letztgenannten Amine können auch aus den genannten Aldehyden oder Ketonen durch Umwandlung nach konventionellen Methoden in ihre Oxime und deren Reduktion mit katalytisch aktiviertem Wasserstoff, vorzugsweise mit Rhodium auf Aluminiumoxyd, erhalten werden.

Die Amid-Ausgangsstoffe des Verfahrens e) können in üblicher Weise aus den genannten primären Aminen der Formel VII und entsprechenden Halogeniden oder Anhydriden von Säuren der Formel $R_1'''-COOH$, worin $R_1'''$ eine Methylengruppe weniger als $R_1$ aufweist, hergestellt werden. Andererseits können diese Ausgangsstoffe durch Umsetzung von 5-Hydroxypyridyl-2-alkansäurehalogeniden oder -anhydriden mit Aminen der Formel $H_2N-R_1$ erhalten werden.

Die erhaltenen Verbindungen der Erfindung können in an sich bekannter Weise ineinander übergeführt werden. So können z. B. erhaltene ungesättigte Verbindungen der allgemeinen Formel I, welche eine Alkenylgruppe $R_1$ enthalten, gleich wie für die Ausgangsstoffe der Formel VI beschrieben, katalytisch hydriert werden.

Erhaltene freie Verbindungen können in entsprechende Säureadditionssalze, z. B. unter Verwendung von anorganischen oder organischen Säuren, vorzugsweise mit Carbonsäuren oder Sulfonsäuren, welche therapeutisch verwendbare Salze ergeben, oder mit entsprechenden Anionenaustauschern, und Isolierung des gewünschten Salzes, umgewandelt werden. Erhaltene Säureadditionssalze können in die entsprechenden freien Verbindungen durch Behandlung mit einer Base, z. B. mit einem Metallhydroxyd, Ammoniak oder einem Hydroxylionenaustauscher übergeführt werden. Säuren, die therapeutisch verwendbare Säureadditionssalze ergeben, sind z. B. anorganische Säuren, wie Halogenwasserstoffsäuren, z. B. Chlorwasserstoff- oder Bromwasserstoffsäure, oder Schwefel-, Phosphor-, Salpeter- oder Perchlorsäure; oder organische Säuren, wie aliphatische oder aromatische Carbon- und Sulfonsäuren, z. B. Ameisen-, Essig-, Propion-, Bernstein-, Glykol-, Milch-, Apfel-, Wein-, Zitronen-, Malein-, Hydroxymalein-, Brenztrauben-, Phenylessig-, Benzoe-, 4-Aminobenzoe-, Anthranil-, 4-Hydroxybenzoe-, Salicyl-, Pamoe-, Nikotin-, Methansulfon-, Äthansulfon-, Hydroxyäthansulfon-, Äthylensulfon-, Benzolsulfon-, Halogenbenzolsulfon-, Toluolsulfon-, Naphthalinsulfon-, Sulfanil-, Cyclohexylsulfaminsäure; oder die Ascorbinsäure.

Diese oder andere Salze, z. B. Pikrate, können auch in der Reinigung von freien Basen verwendet werden. Die Basen werden in ihre Salze übergeführt, die Salze abgetrennt und die Basen aus den Salzen freigesetzt. Infolge der engen Beziehungen zwischen den neuen Verbindungen in freier Form und in Form ihrer Salze sind im vorausgegangenen und nachfolgend unter freien Verbindungen und Salzen sinn- und zweckgemäß gegebenenfalls auch die entsprechenden Salze bzw. freien Verbindungen zu verstehen.

Erhaltene Isomerengemische können nach an sich bekannten Methoden, z. B. durch fraktionierte Destillation, Kristallisation und/oder Chromatographie, in die einzelnen Isomeren getrennt werden. Racemische Produkte können in die optischen Antipoden, z. B. durch Trennung ihrer diastereomeren Salze, z. B. durch fraktionierte Kristallisation der d- oder l-Camphersulfonate oder d- oder l-Mandelate, vorzugsweise solcher Salze der oben genannten $X_1$-Ester, getrennt werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensationsmitteln oder Neutralisationsmitteln, und/oder in einer inerten Atmosphäre, unter Kühlung, bei Zimmertemperatur oder bei erhöhten Temperaturen, bei normalen oder erhöhtem Druck durchgeführt.

Die Erfindung betrifft ebenfalls Abänderungen des vorliegenden Verfahrens, wonach ein auf irgendeiner Stufe des Verfahrens erhältliches Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines Salzes oder eines reaktionsfähigen Derivates verwendet wird. So werden z. B. bei der katalytischen Hydrierung von Carbobenzoxy-Verbindungen der Formel V oder von Schiff'schen Basen VI oder VIa, welche einen olefinischen $R_1$-Rest aufweisen, die entsprechenden gesättigten Verbindungen der Formel I, welche den Alkylrest $R_1$ enthalten, gewonnen.

Beim Verfahren der vorliegenden Erfindung werden vorzugsweise solche Ausgangsstoffe verwendet, welche zu den eingangs als besonders wertvoll geschilderten Verbindungen, insbesondere solchen der Formel II führen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z. B. Laktose, Dextrose, Rohrzucker, Mannitol, Sorbitol, Cellulose und/oder Glycin, und Schmiermitteln, z. B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z. B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, Enzyme der Bindemittel und/oder Brausemischungen, oder Adsorptionsmittel, Farbstoffe, Geschmackstoffe und Süßmittel. Injizierbare Präparate sind vorzugsweise isotonische wäßrige Lösungen oder Suspensionen, und Suppositorien in erster Linie Fettemulsionen oder -suspensionen. Die pharmakologischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologische wertvolle Stoffe enthalten können, werden in an sich bekannter Weise, z. B. mittels konventioneller Misch-, Granulier- oder Dragierverfahren, hergestellt und enthalten von 1% bis 75%, insbesondere von 10% bis 50%, des Aktivstoffes.

Die folgenden Beispiele dienen zur Illustration der Erfindung. Temperaturen werden in Celsiusgraden angegeben und die Angaben über Teile betreffen Gewichtsteile. Wenn nicht anders definiert, wird das Eindampfen von Lösungsmitteln unter vermindertem Druck, z. B. zwischen ungefähr 0,1 und 15 mm Hg, durchgeführt. Das azeotropische Abdampfen wird mit dem beschriebenen Lösungsmittel so lange fortgesetzt, bis ein klares (wasserfreies) Destillat erreicht wird.

## Beispiel 1

Ein Gemisch von 115 g 2-Methyl-5-pyridinol und 1500 ml Tetrahydrofuran wird unter Rühren in einer Stickstoffatmosphäre mit 1350 ml 1,6-molarem n-Butyllithium in Hexan (2,1 Mol) versetzt, wobei man die Temperatur zwischen —15° und 0° hält. Das Reaktionsgemisch wird 1 Stunde bei —10° weiter gerührt, dann mit 114 g Isopropyliminoäthan in solchen Portionen versetzt, daß die Temperatur unter 10° bleibt. Das Gemisch wird 1 Stunde bei Zimmertemperatur gerührt, dann in 2 Teile geteilt und 1 Teil in 750 ml Eiswasser gegossen. Nach gründlichem Schütteln wird die organische Schicht abgetrennt und die wäßrige Schicht mit dem zweiten Teil des obigen Gemisches geschüttelt. Die kombinierten organischen Schichten werden einmal mit 400 ml Wasser extrahiert und verworfen. Alle wäßrigen Lösungen werden vereinigt, dreimal mit 300 ml Diäthyläther gewaschen, mit Chlorwasserstoffsäure auf den pH-Wert 6—6,5 gestellt und viermal mit je 300 ml Essigsäureäthylester gewaschen. Die wäßrige Schicht wird mit wäßriger Natriumhydroxydlösung auf den pH-Wert 8,4 gestellt, mit Natriumchlorid gesättigt und fünfmal mit insgesamt 2300 ml Essigsäureäthylester-Isopropanol (1 : 1) extrahiert. Der Extrakt wird konzentriert, das Konzentrat zweimal mit Isopropanol verdünnt und, um das Wasser azeotropisch zu beseitigen, wieder konzentriert. Die 400 ml des letzten Konzentrats werden filtriert und das Filtrat wird mit Chlorwasserstoff in Essigsäureäthylester auf den pH-Wert 1,5 eingestellt. Der erhaltene Niederschlag wird abfiltriert, mit Isopropanol gewaschen und aus 95%igem wäßrigem Äthanol umkristallisiert. Man erhält das (2-Isopropylaminopropyl)-5-pyridinol-dihydrochlorid, welches bei 210—213° schmilzt.

Der Ausgangsstoff wird wie folgt, frisch hergestellt: Man gibt tropfenweise 170,3 ml Isopropylamin zu 112,5 ml rektifiziertem Acetaldehyd, unter Rühren und Kühlen auf —20 bis —30°. Das Gemisch wird 90 Minuten bei 0° gerührt und dann mit 50 g Kaliumhydroxyd-Tabletten versetzt. Das Gemisch wird in der Kälte belassen und von der verflüssigten Base durch Dekantieren abgetrennt. Weitere 50 g Kaliumhydroxyd-Tabletten werden noch zweimal zugesetzt und das Gemisch wird schließlich im Kühlschrank über Nacht aufbewahrt. Das oben schwimmende Produkt wird abgetrennt, destilliert und die bei 59—62° siedende Fraktion bei atmosphärischem Druck aufgefangen. Man erhält das Isopropyliminoäthan.

## Beispiel 2

Ein Gemisch von 26,7 g 2-(2-Isopropylaminopropyl)-5-pyridinol-dihydrochlorid, 200 ml Methylenchlorid und 100 ml gesättigter wäßriger Natriumcarbonatlösung wird unter Rühren, tropfenweise mit 16 ml Methansulfonylchlorid bei 0—10° versetzt, wobei man das Gemisch durch Zugabe von weiterem Natriumcarbonat basisch hält. Das Gemisch wird 30 Minuten bei einem pH-Wert zwischen 9,5 und 10 weiter gerührt und mit der, der Hälfte seines Volumens entsprechenden Menge gesättigter wäßriger Natriumchloridlösung versetzt. Das Gemisch wird aufgetrennt, die wäßrige Schicht mit Methylenchlorid extrahiert, die vereinigten organischen Lösungen werden mit gesättigter wäßriger Natriumcarbonatlösung gewaschen, getrocknet und eingedampft. Man erhält das 2-(2-Isopropylaminopropyl)-5-me-

thansulfonyloxy-pyridin als ein Öl.

Man löst 39,6 g dieses Öls in 200 ml Isopropanol, versetzt die Lösung mit derjenigen von 11,08 g l-Mandelsäure in 68 ml wasserfreiem Äthanol und läßt das Gemisch bei Zimmertemperatur 20 Stunden stehen. Es wird filtriert, das Filtrat auf 75 ml konzentriert und wieder filtriert. Die vereinigten Rückstände werden aus wasserfreiem Äthanol-Methanol (20 : 3) und dann aus Methanol umkristallisiert. Man erhält das entsprechende l-Mandelat, welches bei 133,5—134° schmilzt.

Die vereinigten Mutterlaugen werden eingedampft und 16 g des Rückstandes in das d-Mandelat, welches bei 131,5—132° schmilzt, umgewandelt.

Man nimmt 11,9 g des l-Mandelats in einer minimalen Menge Wasser auf, stellt die Lösung mit gesättigter wäßriger Natriumcarbonatlösung basisch und extrahiert sie mit Methylenchlorid. Der Extrakt wird getrocknet und eingedampft, wobei man die freie Base erhält. Diese wird in 75 ml Dioxan aufgenommen, mit 62 ml 1-normaler wäßriger Natriumhydroxydlösung versetzt und das Gemisch 2 Stunden unter Rühren unter Rückfluß gekocht. Das Reaktionsgemisch wird konzentriert, das Konzentrat mit Chlorwasserstoffsäure auf den pH-Wert 8,4 eingestellt und mit Natriumchlorid gesättigt. Das Gemisch wird mit Isopropanol-Essigsäureäthylester (1 : 1) extrahiert, der Extrakt azeotropisch eingedampft, der Rückstand in Isopropanol aufgenommen, die Lösung filtriert, mit Chlorwasserstoff in Essigsäureäthylester angesäuert und das Filtrat 2 Tage stehengelassen. Der erhaltene Niederschlag wird abgetrennt und mit Isopropanol gewaschen. Man erhält das linksdrehende 2-(2-Isopropylaminopropyl)-5-pyridinol-dihydrochlorid, welches bei 207—209° schmilzt. $[\alpha]_D^{25} = -11,1°$ (in Wasser).

Der rechtsdrehende Antipode wird ausgehend von oben genanntem d-Mandelat in analoger Weise hergestellt. F. 209—210°. $[\alpha]_D^{25} = +10,0°$ (in Wasser). Diese Verbindung ist pharmakologisch weniger aktiv als das razemische Ausgangsmaterial dieses Beispiels und das genannte linksdrehende Salz.

## Beispiel 3

Eine Lösung, welche aus 6 g 5-Acetoxy-2-pyridylaceton, 100 ml Methanol und 10 ml Isopropylamin hergestellt ist, wird 10 Minuten auf Zimmertemperatur gekühlt und bei dieser Temperatur unter Rühren, innerhalb 20 Minuten mit 4 g Natriumborhydrid portionenweise versetzt. Das Reaktionsgemisch wird eingedampft, der Rückstand in Wasser aufgenommen und die Lösung mit Chloroform gewaschen. Die wäßrige Schicht wird abgetrennt, ihr pH-Wert mit Chlorwasserstoffsäure auf 8,4 eingestellt und mit Natriumchlorid gesättigt. Das Gemisch wird mit Essigsäureäthylester-Isopropanol (1 : 1) extrahiert, der Extrakt durch Zugabe von Isopropanol azeotropisch eingedampft und der Rückstand in Isopropanol aufgenommen. Die Suspension wird filtriert, das Filtrat konzentriert und sein pH-Wert mit Chlorwasserstoff in Essigsäureäthylester auf 1,5 eingestellt. Das Gemisch wird über Nacht im Kühlschrank gehalten, der Niederschlag abgetrennt und mit Isopropanol-Essigsäureäthylester gewaschen. Man erhält das 2-(2-Isopropylaminopropyl)-5-pyridinol-dihydrochlorid, welches bei 207—209° schmilzt. Das Produkt ist mit demjenigen des Beispiels 1 identisch.

Der Ausgangsstoff wird wie folgt hergestellt: Eine Suspension von 32,7 g 2-Methyl-5-pyridinol in 400 ml Tetrahydrofuran wird unter Rühren in einer Stickstoffatmosphäre und Kühlen mit Eis unter 10°, innerhalb 1 Stunde, mit 400 ml 1,6-molarem n-Butyllithium in Hexan versetzt. Das Reaktionsgemisch wird dann mit 29 g Dimethylacetamid in 50 ml Tetrahydrofuran innerhalb 15 Minuten versetzt, dann 2 Stunden bei Zimmertemperatur gerührt und in 700 ml Wasser gegossen. Das Gemisch wird gründlich geschüttelt, die wäßrige Schicht abgetrennt, mit Diäthyläther gewaschen, mit Chlorwasserstoffsäure angesäuert und wieder mit Diäthyläther gewaschen. Der pH-Wert wird mit wäßriger Natriumhydrogencarbonatlösung auf 5 eingestellt und nach Sättigung mit Natriumchlorid wird das Gemisch mit Essigsäureäthylester-Isopropanol (1 : 1) extrahiert. Der Extrakt wird mit Benzol azeotropisch eingedampft und der Rückstand in 100 ml Essigsäureanhydrid aufgenommen. Das Gemisch wird 30 Minuten auf dem Dampfbad gerührt und eingedampft. Der Rückstand wird destilliert und die bei 130—138°/0,9 mm Hg siedende Fraktion aufgefangen. Man erhält das 5-Acetoxy-2-pyridylaceton. Sein Hydrochlorid schmilzt bei 69—72° und das Oxim bei 149—150°.

## Beispiel 4

Eine Suspension von 10,9 g 2-Methyl-5-pyridinol in 200 ml Tetrahydrofuran wird in einer Stickstoffatmosphäre, unter Rühren bei —20°, mit 150 ml 1,6-molarem n-Butyllithium in Hexan innerhalb 30 Minuten versetzt. Nach einer Stunde gibt man bei der genannten Temperatur 25 g 1-Isopropyliminopropan innerhalb 30 Minuten hinzu, rührt das Gemisch 90 Minuten und läßt es auf Zimmertemperatur erwärmen. Das Gemisch wird in 150 ml Wasser gegossen, die organische Schicht mit 50 ml Wasser gewaschen und die vereinigten wäßrigen Lösungen werden mit Diäthyläther gewaschen. Ihr pH-Wert wird zuerst mit Chlorwasserstoffsäure auf 6,8 eingestellt, die Lösung einmal mehr mit Essigsäureäthylester gewaschen, ihr pH-Wert mit wäßriger Natriumcarbonatlösung auf 7,5 erhöht, mit Natriumchlorid gesättigt und mit Essigsäureäthylester-Isopropanol (1 : 1) extrahiert. Der Extrakt wird azeotropisch eingedampft, der Rückstand in Essigsäureäthylester aufgenommen, die Lösung filtriert und mit Chlorwasserstoff in Isopropanol auf den pH-Wert 4,5 eingestellt. Man erhält das 2-(2-Isopropylaminobutyl)-5-pyridinol-hydrochlorid, welches bei 141—143° schmilzt.

Der Ausgangsstoff wird wie folgt hergestellt: Unter Eiskühlung und Rühren versetzt man 58 g Propionaldehyd mit 59 g Isopropylamin und nachfolgend mit 1 ml konzentrierter Chlorwasserstoffsäure. Das Reaktionsgemisch wird 2 Stunden bei Zimmertemperatur gerührt, mit 50 g Kaliumhydroxyd-Tabletten versetzt, 5 Stunden gerührt, von der wäßrigen Phase dekantiert, mit Kaliumhydroxyd getrocknet und destilliert. Die bei 84—86° siedende Fraktion wird aufgefangen. Man erhält das 1-Isopropyliminopropan.

Beispiel 5

Eine Suspension von 9,8 g 2-Methyl-5-pyridinol in 200 ml Tetrahydrofuran wird unter Stickstoff bei —20° gerührt und innerhalb 30 Minuten mit 135 ml 1,6-molarem n-Butyllithium in Hexan versetzt. Nach 3 Stunden wird das Gemisch bei der genannten Temperatur, innerhalb 30 Minuten, mit 10 g tert.-Butyliminoäthan versetzt. Das erhaltene Gemisch wird gemäß Beispiel 4 aufgearbeitet, mit dem Unterschied, daß die letzte Lösung mit Chlorwasserstoff in Essigsäureäthylester angesäuert wird. Man erhält das 2-(2-tert. -Butylaminopropyl)-5-pyridinol-dihydrochlorid, welches bei 218—220° schmilzt.

Beispiel 6

Analog zu den in den vorhergehenden Beispielen beschriebenen Methoden werden auch die folgenden Verbindungen ausgehend von äquivalenten Mengen entsprechender Ausgangsstoffe hergestellt:

a) 2-(3-Isopropylaminobutyl)-5-pyridinol-dihydrochlorid, F. 274—276°;
b) 2-(2-Cyclohexylaminopropyl)-5-pyridinol-dihydrochlorid, F. 232—234°.

Beispiel 7

Eine Suspension von 116 g 2-(2-Isopropylaminopropyl)-5-pyridinol in 310 ml Wasser wird in einer Stickstoffatmosphäre, unter Rühren bei 70°, mit 65 g Fumarsäure versetzt. Die erhaltene klare Lösung wird über Nacht bei 20° gerührt, der erhaltene Niederschlag abfiltriert und dreimal, jeweils mit 15 ml kaltem Wasser gewaschen. Man löst 873 g dieses Niederschlages (erhalten in mehreren Ansätzen) in 2100 ml Wasser bei 70°, filtriert die heiße Lösung und rührt das Filtrat in einer Stickstoffatmosphäre 2 Tage bei Zimmertemperatur. Die erhaltene Suspension wird filtriert und der Rückstand zweimal mit je 250 ml kaltem Wasser gewaschen. Man erhält das 2-(2-Isopropylaminopropyl)-5-pyridinol-monofumarat, welches unter Zersetzung bei 180—183° schmilzt.

Beispiel 8

Ein Gemisch von 342 g 2-Methyl-5-pyridinol und 4500 ml Tetrahydrofuran wird bei einer Temperatur zwischen —18 und —10°, unter Rühren in einer Stickstoffatmosphäre, mit 384,6 g 1,6-molarem n-Butyllithium in Hexan innerhalb 3 Stunden versetzt. Das Gemisch wird eine Stunde bei —10° weiter gerührt, dann innerhalb 5 Minuten mit 340 g Isopropyliminoäthan versetzt und seine Temperatur auf 10° steigen gelassen. Das Reaktionsgemisch wird über Nacht bei Zimmertemperatur gerührt und in 4500 ml Kaltwasser gegossen. Die organische Schicht wird abgetrennt und mit 1200 ml Wasser gewaschen. Die vereinigten wäßrigen Lösungen werden dreimal mit je 900 ml Diäthyläther gewaschen, mit 900 ml konzentrierter Chlorwasserstoffsäure bis zum pH-Wert 6,0 angesäuert und dann mit 853 g Natriumhydrogencarbonat auf den pH-Wert 8,0 eingestellt. Das Gemisch wird bei 60° eingedampft, der Rückstand in 2400 ml Isopropanol bei 60° suspendiert, die Suspension filtriert und das Filtrat bei 60° eingedampft. Der Rückstand wird in 800 ml Wasser aufgenommen, das Gemisch auf 10° gekühlt, filtriert und das Filtrat bei 60° wieder eingedampft.

Man löst 232 g des Niederschlags in 450 ml Äthanol bei 65°, filtriert die heiße Lösung, kühlt das Filtrat auf 27° ab und versetzt es unter Rühren und Kühlen auf 10° mit 380 ml 6-normaler Chlorwasserstoffsäure in Äthanol. Die Suspension wird nach 18 Stunden filtriert und der Rückstand mit 50 ml kaltem Äthanol gewaschen. Man erhält das 2-(2-Isopropylaminopropyl)-5-pyridinol-dihydrochlorid, welches bei 214—216° schmilzt. Das Produkt ist mit demjenigen des Beispiels 1 identisch.

Der Ausgangsstoff wird wie folgt hergestellt: Man kühlt 268,8 g Acetaldehyd auf —25° und versetzt ihn in einer Stickstoffatmosphäre unter Rühren bei dieser Temperatur mit 357,6 Isopropylamin innerhalb 2 Stunden. Das Gemisch wird 2 Stunden bei 0° gerührt und dann mit 150 g Kaliumhydroxyd-Tabletten versetzt. Das Reaktionsgemisch wird 2 Stunden belassen, von der verflüssigten Base durch Dekantieren abgetrennt und mit weiteren 150 g Kaliumhydroxyd behandelt. Das Gemisch wird sodann 2 Stunden bei 0° belassen und auf 150 g Kaliumhydroxyd gegossen; das Reaktionsgemisch wird über Nacht bei 10° aufbewahrt. Das oben schwimmende Produkt wird abgetrennt, bei atmosphärischem Druck destilliert und die bei 59—64° siedende Fraktion aufgefangen. Man erhält das Isoproyliminoäthan.

Beispiel 9

Eine Lösung von 159 g 2-(2-Isopropylaminopropyl)-5-pyridinol-dihydrochlorid in 1000 ml Wasser wird mit 163 g Natriumhydrogencarbonat versetzt und die Suspension eingedampft. Der Rückstand wird in 453 ml wasserfreiem Äthanol bei 60° suspendiert, die Suspension filtriert, der Rückstand dreimal mit je 150 ml wasserfreiem Äthanol gewaschen und die vereinigten Extrakte werden eingedampft. Man erhält das 2-(2-Isopropylaminopropyl)-5-pyridinol, welches bei 135—138° schmilzt.

Beispiel 10

Eine Lösung von 1,55 g 5-Hydroxy-2-pyridylaceton in 50 ml Methanol wird unter Rühren bei Zimmertemperatur mit 0,61 g 2-Propenylamin, 2 ml 5,5-normalem ätherischem Chlorwasserstoff und 0,5 g Natriumcyanborhydrid in dieser Reihenfolge versetzt. Das Gemisch wird sieben Tage bei Zimmertemperatur gerührt und sein pH-Wert dann durch vorsichtige Zugabe von 2-normaler Chlorwasserstoffsäure auf 1 eingestellt. Das saure Gemisch wird eingedampft, der Rückstand in 20 ml Wasser gelöst und der pH-Wert der Lösung mit festem Natriumhydrogencarbonat auf 8 eingestellt. Das Gemisch wird eingedampft, der Rückstand mit Isopropanol trituriert und in 25 ml Aceton aufgelöst. Die Lösung wird filtriert, mit 1,28 g Fumarsäure in einer minimalen Menge heißem Aceton versetzt und das erhaltene feste Material abfiltriert. Man erhält das 2-[2-(2-Propenylamino)-propyl]-5-pyridinol-fumarat, welches bei 194—195° schmilzt.

Der Ausgangsstoff wird wie folgt hergestellt: Ein Gemisch aus 103,6 g 5-Acetoxypyridyl-2-aceton, 0,75 g wasserfreiem Kaliumcarbonat und 400 ml wasserfreiem Äthanol wird 24 Stunden unter Rückfluß gekocht, filtriert, auf 100 ml konzentriert, das Konzentrat gekühlt und der Niederschlag abgetrennt. Man erhält das 5-Hydroxy-2-pyridylaceton, welches bei 119—120° schmilzt.

Beispiel 11

Eine Lösung von 0,8 g 5-Hydroxy-2-pyridylaceton in 25 ml Methanol wird mit 0,33 g Cyclopropylamin, 1 ml 5-normalem ätherischem Chlorwasserstoff und 1,16 g Natriumcyanborhydrid in dieser Reihenfolge versetzt und das Gemisch 3 Tage bei Zimmertemperatur gerührt. Sein pH-Wert wird dann mit 5-normalem ätherischem Chlorwasserstoff auf 1 und nachher mit festem Natriumhydrogencarbonat auf 8 eingestellt. Das Gemisch wird filtriert, eingedampft, der Rückstand auf Silicagel chromatographiert und mit Essigsäureäthylester-Methanol (4 : 1) eluiert. Man erhält das 2-(2-Cyclopropylaminopropyl)-5-pyridinol. Das Produkt wird gemäß Beispiel 7 in sein Monofumarat umgewandelt, welches bei 161° unter Zersetzung schmilzt.

Beispiel 12

Eine Lösung von 1,62 g 2-(2-Aminopropyl) -5-pyridinol-hydrochlorid in 50 ml Methanol wird zuerst mit 0,83 g n-Hexanol, dann mit 1,75 g Natriumcyanborhydrid versetzt und das Gemisch 3 Tage bei Zimmertemperatur gerührt. Sein pH-Wert wird zuerst mit 5-normalem ätherischem Chlorwasserstoff auf 1 und dann mit festem Natriumhydrogencarbonat auf 8 eingestellt. Das Gemisch wird filtriert, eingedampft und der Rückstand mit Isopropanol trituriert. Man erhält das ölige 2-(2-n-Hexylaminopropyl)-5-pyrdinol, welches im Massenspektrum Spitzen bei 151, 128 und 109 m/e zeigt.

Der Ausgangsstoff wird wie folgt hergestellt: Ein Gemisch von 8,64 g 5-Hydroxy-2-pyridylaceton, 4,08 g Hydroxylaminhydrochlorid und 170 ml wasserfreiem Äthanol wird 64 Stunden unter Rückfluß gekocht und eingedampft. Der Rückstand wird in einer minimalen Menge Äthanol aufgenommen, die Lösung filtriert und das Filtrat eingedampft. Man erhält das 2-(2-Oximino-propyl)-5-pyridinol-hydrochlorid, welches bei 141—145° schmilzt.

Ein Gemisch von 12 g der letztgenannten Verbindung, 500 ml mit Ammoniak gesättigtes Methanol und 2,4 g Rhodium auf Aluminiumoxyd wird bei Zimmertemperatur und atmosphärischem Druck 3 Wochen hydriert. Das Gemisch wird filtriert und das Filtrat eingedampft. Man erhält das 2-(2-Aminopropyl)-5-pyridinol-hydrochlorid, welches ohne weitere Reinigung verwendet wird.

Eine geringe Menge der letztgenannten Verbindung wird mit Fumarsäure angesäuert und der Niederschlag aus Essigsäureäthylester-Äthanol umkristallisiert. Man erhält das entsprechende Fumarat,welches bei 171—176° schmilzt.

Beispiel 13

Eine Lösung von 0,27 g 2-(2-Aminopropyl)-5-pyridinol-dihydrochlorid und 0,5 ml Diisopropyl-äthylamin in 0,5 ml Methanol wird unter Rühren bei Zimmertemperatur mit 0,24 g Isopropyljodid versetzt. Nach 24 Stunden wird das Gemisch eingedampft und der Rückstand gemäß Beispiel 9 in das 2-(2-Isopropylaminopropyl)-5-pyridinol, welches bei 135—138° schmilzt, umgewandelt. Die beiden freien Basen sind identisch.

Der Ausgangsstoff wird wie folgt hergestellt: Eine Lösung von 0,19 g 2-(2-Aminopropyl)-5-pyridinol-

hydrochlorid in 10 ml wasserfreiem Äthanol wird mit 0,2 ml ätherischem Chlorwasserstoff versetzt und eingedampft. Man erhält das 2-(2-Aminopropyl)-5-pyridinol-dihydrochlorid, welches bei 125—128° unter Zersetzung schmilzt.

Beispiel 14

Eine Lösung von 0,1 g 2-(2-acetylamino-propyl)-5-pyridinol in 10 ml wasserfreiem Tetrahyrofuran wird unter Rühren bei 0° mit 1,5 ml 1-molarer Lösung von Alan-triäthylamin in Toluol tropfenweise versetzt. Das Gemisch wird nach 12 Stunden mit 10 ml 2-normaler wäßriger Natriumhydroxydlösung bei 0° versetzt und mit Isopropanol azeotropisch eingedampft. Der Rückstand wird in 25 ml Methanol aufgenommen, der pH-Wert der Lösung mit 5-normalem ätherischem Chlorwasserstoff auf 8 eingestellt, die erhaltenen Salze werden abfiltriert und das Filtrat wird eingedampft. Der Rückstand wird mit Isopropanol trituriert. Man erhält das 2-(2-Äthylamino-propyl)-5-pyridinol, welches bei 75—79° schmilzt.

Der Ausgangsstoff wird wie folgt hergestellt: Eine Suspension von 1,01 g 2-(2-Aminopropyl)-5-pyridinol-hydrochlorid in 40 ml Methylenchlorid wird unter Rühren bei Zimmertemperatur zuerst mit 3,3 ml Pyridin und dann mit 1,47 g Acetylchlorid versetzt. Nach 17 Stunden wird das Reaktionsgemisch mit dem gleichen Volumen gesättigter wäßriger Natriumhydrogencarbonatlösung versetzt, die organische Lösung abgetrennt, getrocknet und eingedampft. Das als Rückstand erhaltene Öl wird auf Silicagel chromatographiert und mit Methanol-Essigsäureäthylester (4 : 1) eluiert. Man erhält das 5-Acetoxy-2-(2-acetylaminopropyl)-pyridin, welches im IR-Spektrum Banden bei 1578 und 1658 $cm^{-1}$ zeigt. Es wird alkoholysiert wie folgt: Eine Lösung von 240 mg der letztgenannten Verbindung in 5 ml wasserfreiem Äthanol, welches 7,5 mg wasserfreies Kaliumcarbonat enthält, wird 5 Stunden unter Rückfluß gekocht. Man erhält das 2-(2-Acetylaminopropyl)-5-pyridinol, welches ohne weitere Reinigung verwendet wird.

Beispiel 15

Eine Lösung von 0,15 g Dibenzoyl-2-(2-methylaminopropyl)-5-pyridinol in 5 ml 5-normaler Chlorwasserstoffsäure wird 23 Stunden unter Rückfluß gekocht, abgekühlt, zweimal mit 5 ml Diäthyläther gewaschen und eingedampft. Man erhält das 2-(2-Methylaminopropyl)-5-pyridinol-dihydrochlorid, welches im Massenspektrum Spitzen bei 165, 151, 109 und 58 m/e zeigt.

Der Ausgangsstoff wird wie folgt hergestellt: Ein Gemisch von 0,5 g 2-(2-Aminopropyl)-5-pyridinol-hydrochlorid, 0,85 g Benzoylchlorid, 10 ml Methylenchlorid und 10 ml gesättigter wäßriger Natriumhydrogencarbonatlösung wird 1 Stunde bei Zimmertemperatur gerührt. Die organische Phase wird abgetrennt, getrocknet und eingedampft. Der Rückstand wird aus Diäthyläther umkristallisiert. Man erhält das Dibenzoyl-2-(2-aminopropyl)-5-pyridinol, welches bei 110—112° schmilzt.

Man löst 0,36 g der letztgenannten Verbindung in 1,5 ml Dimethylformamid und gibt die Lösung bei Zimmertemperatur zu der Aufschlämmung von 36 mg Natriumhydrid in 1 ml Dimethylformamid. Das Gemisch wird 15 Minuten erhitzt, in einer Stunde auf Zimmertemperatur gebracht und dann auf 0° gekühlt. Das Gemisch wird mit 1 ml Toluol verdünnt und mit 0,36 g Methyliodid schnell versetzt. Das Reaktionsgemisch wird bei Zimmertemperatur 1 Stunde gerührt und dann mit 20 ml Wasser versetzt. Es wird zweimal mit 20 ml Diäthyläther extrahiert und die Extrakte werden eingedampft. Man erhält das ölige Dibenzoyl-2-(2-methylaminopropyl)-5-pyridinol, welches im NMR-Spektrum eine Bande bei 3,48 ppm zeigt.

Man kann 0,15 g der letztgenannten Verbindung in 4 ml wasserfreiem Methanol in Gegenwart von 0,15 mg Kaliumcarbonat, unter 12stündigem Rühren bei Zimmertemperatur, partiell alkoholysieren, wobei man das 2-(N-Benzoyl-2-methylaminopropyl)-5-pyridinol erhält. Dieses kann den oben genannten Dibenzoyl-Ausgangsstoff in der sauren Hydrolyse ersetzen.

In analoger Weise wird ein Gemisch von 0,1 g Dibenzoyl-2-(2-Isopropylaminopropyl)-5-pyridinol und 10 ml 5-normaler Chlorwasserstoffsäure 3 Tage unter Rückfluß gekocht, abgekühlt, filtriert, eingedampft und der Rückstand mit Aceton trituriert. Man erhält das 2-(2-Isopropylaminopropyl)-5-pyridinol-dihydrochlorid, welches bei 199—204° schmilzt. Das Produkt ist mit demjenigen der Beispiele 1, 3 und 8 identisch.

Beispiel 16

Ein Gemisch von 0,08 g 2-(N-Carbobenzyloxy-2-methylaminopropyl)-5-pyridinol und 2,5 ml 5-normaler Chlorwasserstoffsäure wird 23 Stunden unter Rückfluß gekocht, abgekühlt und mit Diäthyläther extrahiert. Der Extrakt wird getrocknet und eingedampft. Man erhält das 2-(2-Methylaminopropyl)-5-pyridinol-dihydrochlorid, welches mit dem Produkt des Beispiels 15 identisch ist.

Der Ausgangsstoff wird wie folgt hergestellt: Eine Lösung von 0,5 g 2-(2-Aminopropyl)-5-pyridinol in 10 ml Methylenchlorid und 10 ml gesättigter wäßriger Natriumhydrogencarbonatlösung wird unter Rühren bei Zimmertemperatur mit 1,04 g Benzyloxycarbonylchlorid versetzt. Nach 12 Stunden wird die organische Phase abgetrennt, getrocknet, eingedampft und der Rückstand aus Diäthyläther-Hexan

umkristallisiert. Man erhält das Bis-carbobenzyloxy-2-(2-aminopropyl)-5-pyridinol, welches bei 63—65° schmilzt.

Eine Lösung von 0,42 g der letztgenannten Verbindung in 2 ml Dimethylformamid wird zu der Aufschlämmung von 36 mg Natriumhydrid in 1,5 ml Dimethylformamid gegeben und das Gemisch eine Stunde auf 55° erhitzt. Das Gemisch wird in 4 Stunden auf 25° gebracht, auf 0° gekühlt, mit 1 ml Toluol verdünnt und mit 0,36 g Methyljodid versetzt. Das Gemisch wird bei Zimmertemperatur 12 Stunden gerührt und dann mit 10 ml Dinatriumphosphat-Puffer versetzt. Die wäßrige Phase wird zweimal mit 20 ml Diäthyläther extrahiert, der Extakt mit Wasser gewaschen, getrocknet und eingedampft. Man erhält das Bis-carbobenzyloxy-2-(2-methylaminopropyl)-5-pyridinol und das 2-(N-Carbobenzyloxy-2-methylaminopropyl)-5-pyridinol in ungefähr gleichen Mengen. Dieses Gemisch wird in Essigsäureäthylester aufgenommen, die Lösung mit 1-normaler wäßriger Natriumhydroxydlösung extrahiert und die wäßrige Phase abgetrennt. Ihr pH-Wert wird mit Mononatriumphosphat-Puffer auf 8 eingestellt. Das Gemisch wird mit Essigsäureäthylester extrahiert, der Extrakt getrocknet und eingedampft. Man erhält das 2-(N-Carbobenzyloxy-2-methylaminopropyl)-5-pyridinol.

Beispiel 17

Ein Gemisch von 0,08 g 2-(N-Carbobenzyloxy-2-methylaminopropyl)-5-pyridinol, 5 ml mit wasserfreiem Chlorwasserstoff gesättigtes wasserfreies Äthanol und 0,04 g Palladium-auf-Kohle-Katalysator wird 18 Stunden bei Zimmertemperatur und atmosphärischem Druck hydriert. Das Gemisch wird filtriert, der Rückstand mit äthanolischem Chlorwasserstoff gewaschen und das Filtrat eingedampft. Man erhält das 2-(2-Methylaminopropyl)-5-pyridinol-dihydrochlorid, welches mit dem Produkt der Beispiele 15 und 16 identisch ist.

Beispiel 18

Herstellung von 10 000 Tabletten mit einem Gehalt von je 100 mg der aktiven Substanz:

Bestandteile:

| | |
|---|---|
| 2-(2-Isopropylaminopropyl)-5-pyridinol-dihydrochlorid | 1000 g |
| Milchzucker | 2535 g |
| Maisstärke | 125 g |
| Polyäthylenglykol 6000 | 150 g |
| Talkpulver | 150 g |
| Magnesiumstearat | 40 g |
| Gereinigtes Wasser | q. s. |

Verfahren

Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 65 ml Wasser suspendiert und die Suspension zur siedenden Lösung von Polyäthylenglykol in 260 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 10,3 mm Durchmesser, welche eine Bruchrille aufweisen, gepreßt.

Beispiel 19

Herstellung von 1000 Kapseln mit einem Gehalt von je 50 mg der aktiven Substanz:

Bestandteile:

| | |
|---|---|
| 2-(2-Isopropylaminopropyl)-5-pyrydinol-monofumarat | 50 g |
| Maisstärke | 5 g |
| Milchzucker | 143,75 g |
| Magnesiumstearat | 1 g |
| Netzmittel | 0,25 g |

Verfahren

Sämtliche pulverigen Bestandteile werden mit einem Sieb von 0,6 mm Maschenweite gesiebt. Dann wird der Wirkstoff zuerst mit Magnesiumstearat und Netzmittel und darauffolgend mit Stärke und

Milchzucker in einem geeigneten Mischer homogenisiert. Kapseln Nr. 2 werden mit je 200 mg des Gemisches mit einer Füllmaschine gefüllt.

In analoger Weise werden Tabletten oder Kapseln, welche eine andere Verbindung der Erfindung, z. B. eine solche der vorhergehenden Beispiele enthalten, hergestellt.

**Patentansprüche für die Vertragsstaaten: BE, CH, DE, FR, IT, LI, LU, NL, SE**

1. Sekundäre 2-Aminoalkyl-5-pyridinole der allgemeinen Formel I

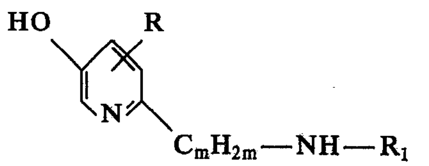

(I)

worin R Wasserstoff oder Alkyl mit 1—4 Kohlenstoffatomen bedeutet, m für eine ganze Zahl von 2 bis 4 steht, und $R_1$ Alkyl, Cycloalkyl oder Alkenyl mit jeweils höchstens 7 Kohlenstoffatomen bedeutet, wobei sich in Alkenylresten die Doppelbindung in 2- oder 3-Stellung befindet, und ihre Säureadditionssalze.

2. Verbindungen gemäß Anspruch 1, worin R Wasserstoff oder Methyl bedeutet, und die anderen Symbole die im Anspruch 1 angegebenen Bedeutungen haben.

3. Verbindungen gemäß Anspruch 1, worin R Wasserstoff oder Methyl bedeutet, m für die ganze Zahl 2 oder 3 steht, und $R_1$ Alkyl mit 2 bis 6 C-Atomen oder Cycloalkyl oder Alkenyl, mit jeweils 3 bis 6 C-Atomen bedeutet, wobei sich in Alkenylresten die Doppelbindung in 2- oder 3-Stellung befindet.

4. Verbindungen der allgemeinen Formel II

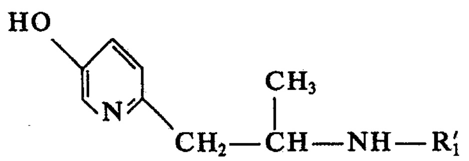

(II)

worin $R_1'$ Alkyl, Cycloalkyl oder Alkenyl mit jeweils 3 bis 6 C-Atomen bedeutet, wobei sich in Alkenylresten die Doppelbindung in 2- oder 3-Stellung befindet, und ihre Säureadditionssalze.

5. Verbindungen gemäß Anspruch 4, worin $R_1'$ Isopropyl oder tert.-Butyl bedeutet.

6. Verbindungen gemäß Anspruch 4, worin $R_1'$ Isopropyl, tert.-Butyl, Allyl oder Cyclopropyl bedeutet.

7. 2-(2-Isopropylaminopropyl)-5-pyridinol und seine Säureadditionssalze.

8. Der linksdrehende Antipode der in den Ansprüchen 1 und 6 beanspruchten Verbindungen.

9. Der linksdrehende Antipode der in den Ansprüchen 2 bis 5 und 7 beanspruchten Verbindungen.

10. Verbindungen gemäß den Ansprüchen 1, 6 und 8 zur Verwendung als kardioprotektive Mittel.

11. Verbindungen gemäß den Ansprüchen 2 bis 5, 7 und 9 zur Verwendung als kardioprotektive Mittel.

12. Pharmazeutische Präparate, enthaltend Verbindungen gemäß einem der Ansprüche 1, 6, 8 und 10 oder therapeutisch verwendbare Säureadditionssalze von solchen Verbindungen.

13. Pharmazeutische Präparate, enthaltend Verbindungen gemäß einem der Ansprüche 2 bis 5, 7, 9 und 11 oder therapeutisch verwendbare Säureadditionssalze von solchen Verbindungen.

14. Die Verbindungen der Ansprüche 1, 6, 8 und 10 zur Anwendung in einem Verfahren zur therapeutischen Behandlung des menschlichen oder tierischen Körpers.

15. Die Verbindungen der Ansprüche 2 bis 5, 7, 9 und 11 zur Anwendung in einem Verfahren zur therapeutischen Behandlung des menschlichen oder tierischen Körpers.

16. Verfahren zur Herstellung von sekundären 2-Aminoalkyl-5-pyridinolen gemäß Anspruch 1, dadurch gekennzeichnet, daß man

a) an eine Verbindung der allgemeinen Formel III

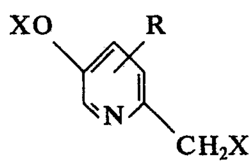

(III)

worin X ein Alkalimetallatom oder eine Erdalkalimetall-Halogengruppe bedeutet, eine Verbindung der allgemeinen Formel IV

$$C_{m-1}H_{2m-2}{=}N{-}R_1 \qquad (IV)$$

addiert und das Produkt der allgemeinen Formel I aus dem erhaltenen Metallsalz freisetzt, oder

b) in einer Verbindung der allgemeinen Formel V

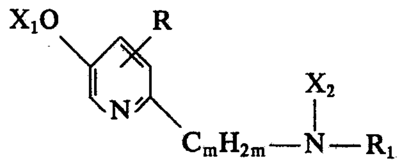

(V)

worin jedes der Symbole $X_1$ und $X_2$ Wasserstoff oder das Acylradikal einer aliphatischen oder aromatischen Carbonsäure oder Sulfonsäure bedeutet, mit der Maßgabe, daß mindestens eines dieser Symbole für ein Acylradikal steht, das Acylradikal $X_1$ und/oder $X_2$ durch Solvolyse oder Hydrogenolyse durch Wasserstoff ersetzt, oder

c) eine Schiff'sche Base der allgemeinen Formel VI oder VIa

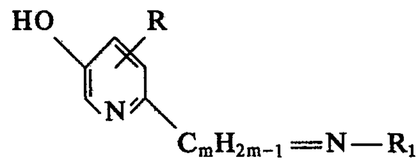

(VI)

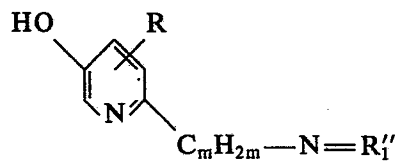

(VIa)

wobei das mit dem Stickstoffatom verbundene Kohlenstoffatom des Restes $R_1''$ ein Wasserstoffatom weniger als $R_1$ aufweist, reduziert, oder

d) ein primäres Amin der allgemeinen Formel VII

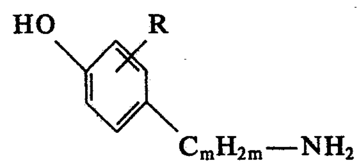

(VII)

mit einem reaktionsfähigen Ester des Alkohols $R_1{-}OH$ in Gegenwart einer starken Base kondensiert, oder

e) ein einem Amin der allgemeinen Formel I entsprechendes Amid, worin ein der sekundären Aminogruppe benachbartes Kohlenstoffatom anstelle von zwei Wasserstoffatomen ein Sauerstoffatom aufweist, reduziert und, wenn erwünscht, eine erhaltene Verbindung in eine andere Verbindung der Erfindung umwandelt, und/oder, wenn erwünscht, eine erhaltene Base in ein Säureadditionssalz oder ein erhaltenes Salz in die freie Base oder in ein anderes Säureadditionssalz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

17. Die nach dem Verfahren des Anspruchs 16 erhältlichen Verbindungen.

**Patentansprüche für den Vertragsstaat: AT**

1. Verfahren zur Herstellung von sekundären 2-Aminoalkyl-5-pyridinolen der allgemeinen Formel I

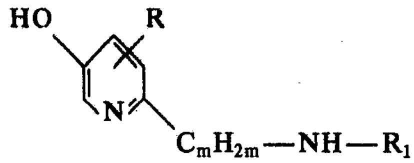

(I)

worin R Wasserstoff oder Alkyl mit 1—4 Kohlenstoffatomen bedeutet, m für eine ganze Zahl von 2 bis 4 steht, und $R_1$ Alkyl, Cycloalkyl oder Alkenyl mit jeweils höchstens 7 Kohlenstoffatomen bedeutet, wobei sich in Alkenylresten die Doppelbindung in 2- oder 3-Stellung befindet, und ihren Säureadditionssalzen, dadurch gekennzeichnet, daß man

a) an eine Verbindung der allgemeinen Formel III

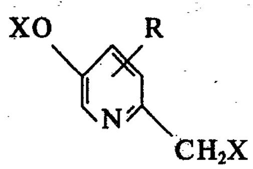

(III)

worin X ein Alkalimetallatom oder eine Erdalkalimetall-Halogengruppe bedeutet, eine Verbindung der allgmeinen Formel IV

$$C_{m-1}H_{2m-2}{=}N{-}R_1 \quad (IV)$$

addiert und das Produkt der allgemeinen Formel I aus dem erhaltenen Metallsalz freisetzt, oder

b) in einer Verbindung der allgemeinen Formel V

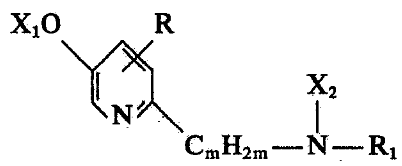

(V)

worin jedes der Symbole $X_1$ und $X_2$ Wasserstoff oder das Acylradikal einer aliphatischen oder aromatischen Carbonsäure oder Sulfonsäure bedeutet, mit der Maßgabe, daß mindestens eines dieser Symbole für ein Acylradikal steht, das Acylradikal $X_1$ und/oder $X_2$ durch Solvolyse oder Hydrogenolyse durch Wasserstoff ersetzt, oder

c) eine Schiff'sche Base der allgemeinen Formel VI oder VIa

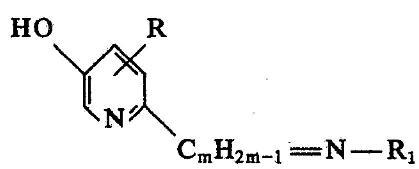

(VI)

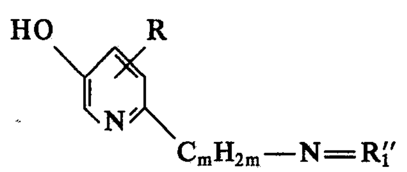

(VIa)

wobei das mit dem Stickstoffatom verbundene Kohlenstoffatom des Restes $R_1''$ ein Wasserstoffatom weniger als $R_1$ aufweist, reduziert, oder

d) ein primäres Amin der allgemeinen Formel VII

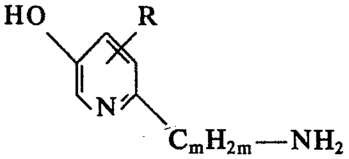

(VII)

mit einem reaktionsfähigen Ester des Alkohols $R_1$—OH in Gegenwart einer starken Base kondensiert, oder

e) ein einem Amin der allgemeinen Formel I entsprechendes Amid, worin ein der sekundären Aminogruppe benachbartes Kohlenstoffatom anstelle von zwei Wasserstoffatomen ein Sauerstoffatom aufweist, reduziert und, wenn erwünscht, eine erhaltene Verbindung in eine andere Verbindung der Erfindung umwandelt, und/oder, wenn erwünscht, eine erhaltene Base in ein Säureadditionssalz oder ein erhaltenes Salz in die freie Base oder in ein anderes Säureadditionssalz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

2. Verfahren nach Anspruch 1, dadurch gekennzeichnet, daß man in der Reaktion a) Ausgangsstoffe der Formel III verwendet, in welchen X Lithium, Natrium oder Halogenmagnesium bedeutet.

3. Verfahren nach Anspruch 1, dadurch gekennzeichnet, daß man in der Reaktion b) das Acylradikal $X_1$ und/oder $X_2$ durch Behandlung mit solvolysierenden oder hydrogenolysierenden Mitteln durch Wasserstoff ersetzt.

4. Verfahren nach Anspruch 1, dadurch gekennzeichnet, daß man in der Reaktion c) die Reduktion mit katalytisch aktiviertem oder nascierendem Wasserstoff, oder mit einfachen oder komplexen Leichtmetallhydriden durchführt.

5. Verfahren nach Anspruch 1, dadurch gekennzeichnet, daß man in der Reaktion d) solche Ausgangsstoffe verwendet, in welcher der reaktionsfähige Ester von starken anorganischen Säuren oder organischen Sulfonsäuren abgeleitet ist.

6. Verfahren nach Anspruch 1, dadurch gekennzeichnet, daß man in der Reaktion e) die Reduktion mit einfachen oder komplexen Leichtmetallhydrid durchführt.

7. Verfahren nach einem der Ansprüche 1 bis 6, dadurch gekennzeichnet, daß man Verbindungen der allgemeinen Formel I, worin R Wasserstoff oder Methyl bedeutet, m für die ganze Zahl 2 oder 3 steht, und $R_1$ Alkyl mit 2 bis 6 C-Atomen oder Cycloalkyl oder Alkenyl mit jeweils 3 bis 6 C-Atomen bedeutet, wobei sich in Alkenylresten die Doppelbindung in 2- oder 3-Stellung befindet, und ihre Säureadditionssalze herstellt.

8. Verfahren nach einem der Ansprüche 1 bis 6, dadurch gekennzeichnet, daß man Verbindungen der allgemeinen Formel II

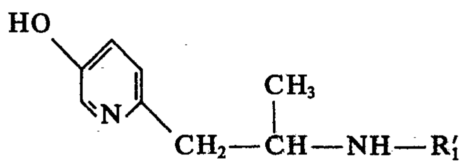

(II)

worin $R_1'$ Alkyl, Cycloalkyl oder Alkenyl mit jeweils 3 bis 6 C-Atomen bedeutet, wobei sich in Alkenylresten die Doppelbindung in 2- oder 3-Stellung befindet, und ihre Säureadditionssalze herstellt.

9. Verfahren nach einem der Ansprüche 1 bis 6, dadurch gekennzeichnet, daß man Verbindungen der Formel II, worin $R_1'$ i-Propyl, tert.-Butyl, Allyl oder Cyclopropyl bedeutet, und ihre Säureadditionssalze herstellt.

10. Verfahren nach einem der Ansprüche 1 bis 6, dadurch gekennzeichnet, daß man 2-(2-Isopropylaminopropyl)-5-pyridinol und seine Säureadditionssalze herstellt.

11. Verfahren nach einem der Ansprüche 1 bis 10, dadurch gekennzeichnet, daß man die erhaltenen Verfahrensprodukte in der Form ihrer linksdrehenden Antipoden isoliert.

12. Verfahren nach Anspruch 1 zur Herstellung von 2-Aminoalkyl-5-pyridinolen der im Anspruch 1 gezeigten allgemeinen Formel I, worin R Wasserstoff oder Methyl bedeutet, und die anderen Symbole die im Anspruch 1 angegebenen Bedeutungen haben, und ihren Säureadditionssalzen, dadurch gekennzeichnet, daß man

a) an eine Verbindung der im Anspruch 1 gezeigten Formel III, worin R Wasserstoff oder Methyl bedeutet, und X für ein Alkalimetallatom steht, eine Verbindung der im Anspruch 1 gezeigten Formel IV addiert und das Verfahrensprodukt aus dem erhaltenen Metallsalz freisetzt, oder

b) eine Schiff'sche Base der im Anspruch 1 gezeigten Formel VI, worin R Wasserstoff oder Methyl bedeutet, reduziert, und wenn erwünscht, eine erhaltene Base in ein Säureadditionssalz oder ein

erhaltenes Salz in die freie Base oder in ein anderes Säureadditionssalz überführt, und/oder, wenn erwünscht, ein erhaltenes Gemisch von Isomeren oder Racematen in die einzelnen Isomeren oder Racemate auftrennt, und/oder, wenn erwünscht, erhaltene Racemate in die optischen Antipoden aufspaltet.

13. Verfahren nach Anspruch 12, dadurch gekennzeichnet, daß man in der Reaktion a) die Freisetzung des Verfahrensprodukts mit Wasser oder verdünnten anorganischen oder organischen Säuren durchführt.

14. Verfahren nach Anspruch 12, dadurch gekennzeichnet, daß man in der Reaktion b) die Reduktion mit katalytisch aktiviertem oder nascierendem Wasserstoff, oder mit einfachen oder komplexen Leichtmetallhydriden durchführt.

15. Verfahren nach einem der Ansprüche 12 bis 14, dadurch gekennzeichnet, daß man Verbindungen der allgemeinen Formel I, worin R Wasserstoff oder Methyl bedeutet, m für die ganze Zahl 2 oder 3 steht, $R_1$ Alkyl mit 2 bis 6 C-Atomen oder Cycloalkyl oder Alkenyl mit jeweils 3 bis 6 C-Atomen bedeutet, wobei sich in Alkenylresten die Doppelbindung in 2- oder 3-Stellung befindet, und ihre Säureadditionssalze herstellt.

16. Verfahren nach einem der Ansprüche 12 bis 14, dadurch gekennzeichnet, daß man Verbindungen der im Anspruch 8 gezeigten Formel II, worin $R_1'$ Alkyl, Cycloalkyl oder Alkenyl mit jeweils 3 bis 6 C-Atomen bedeutet, wobei sich in Alkenylresten die Doppelbindung in 2- oder 3-Stellung befindet, und ihre Säureadditionssalze herstellt.

17. Verfahren nach einem der Ansprüche 12 bis 14, dadurch gekennzeichnet, daß man Verbindungen der im Anspruch 8 gezeigten Formel II, worin $R_1'$ Isopropyl oder tert.-Butyl bedeutet, und ihre Säureadditionssalze herstellt.

18. Verfahren nach einem der Ansprüche 12 bis 14, dadurch gekennzeichnet, daß man 2-(2-Isopropylaminopropyl)-5-pyridinol und seine Säureadditionssalze herstellt.

19. Verfahren nach einem der Ansprüche 12 bis 18, dadurch gekennzeichnet, daß man die erhaltenen Verfahrensprodukte in der Form ihrer linksdrehenden Antipoden isoliert.

20. Verfahren zur Herstellung eines pharmazeutischen Präparates, gekennzeichnet durch die Verarbeitung eines erfindungsgemäßen Wirkstoffs nach einem der Ansprüche 1 bis 11, mit einem pharmazeutischen Trägermaterial.

21. Verfahren zur Herstellung eines pharmazeutischen Präparates, gekennzeichnet durch die Verarbeitung eines erfindungsgemäßen Wirkstoffs nach einem der Ansprüche 12 bis 19, mit einem pharmazeutischen Trägermaterial.

**Claims for the Contracting States: BE, CH, DE, FR, IT, LI, LU, NL and SE**

1. Secondary 2-aminoalkyl-5-pyridinols of the general formula I

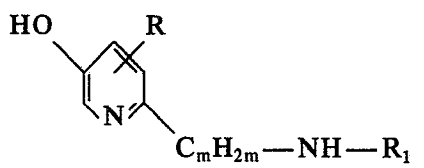

(I)

wherein R is hydrogen or alkyl having 1—4 carbon atoms, m is an integer from 2 to 4, and $R_1$ is alkyl, cycloalkyl or alkenyl having in each case at most 7 carbon atoms, the double bond in alkenyl radicals being in the 2- or 3-position; and acid addition salts thereof.

2. Compounds according to claim 1, wherein R is hydrogen or methyl, and the other symbols have the meanings defined in claim 1.

3. Compounds according to claim 1, wherein R is hydrogen or methyl, m is the integer 2 or 3, and $R_1$ is alkyl having 2 to 6 C atoms or cycloalkyl or alkenyl having in each case 3 to 6 C atoms, the double bond in alkenyl radicals being in the 2 or 3-position.

4. Compounds of the general formula II

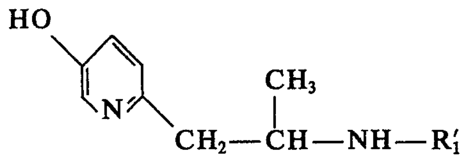

(II)

wherein $R_1'$ is alkyl, cycloalkyl or alkenyl each having 3 to 6 C atoms, the double bond in alkenyl radicals being in the 2- or 3-position; and acid addition salts thereof.

5. Compounds according to claim 4, wherein $R_1'$ is isopropyl or tert-butyl.

6. Compounds according to claim 4, wherein $R_1'$ is isopropyl, tert-butyl, allyl or cyclopropyl.

7. 2-(2-Isopropylaminopropyl)-5-pyridinol and acid addition salts thereof.

8. The levorotatory optical antipode of the compounds claimed in claims 1 and 6.

9. The levorotatory optical antipode of the compounds claimed in claims 2 to 5 and 7.

10. Compounds according to claims 1, 6 and 8 for use as cardioprotective agents.

11. Compounds according to claims 2 to 5, 7 and 9 for use as cardioprotective agents.

12. Pharmaceutical preparations containing compounds according to one of claims 1, 6, 8 and 10, or therapeutically applicable acid addition salts of such compounds.

13. Pharmaceutical preparations containing compounds according to one of claims 2 to 6, 7, 9 and 11 or therapeutically applicable acid addition salts of such compounds.

14. The compounds of claims 1, 6, 8 and 10 for use in a process for the therapeutic treatment of the human or animal body.

15. The compounds of claims 2 to 5, 7, 9 and 11 for use in a process for the therapeutic treatment of the human or animal body.

16. Process for producing secondary 2-aminoalkyl-5-pyridinols according to claim 1, characterised in that

a) to a compound of the general formula III

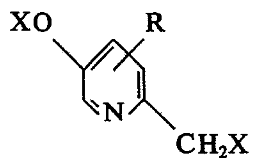

(III)

wherein X is an alkali metal atom or an alkaline-earth metal halogen group, there is added, by an addition reaction, a compound of the general formula IV

$$C_{m-1}H_{2m-2}=N-R_1 \qquad \text{(IV)}$$

and the product of the general formula I is liberated from the metal salt obtained; or

b) in a compound of the general formula V

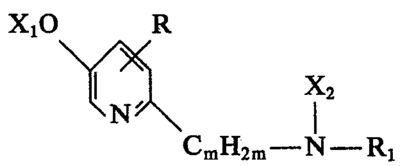

(V)

wherein each of the symbols $X_1$ and $X_2$ is hydrogen or the acyl radical of an aliphatic or aromatic carboxylic acid or sulfonic acid, with the proviso that at least one of these symbols is an acyl radical, the acyl radical $X_1$ and/or $X_2$ is replaced, by solvolysis or hydrogenolysis, by hydrogen; or

c) a Schiff base of the general formula VI or VIa

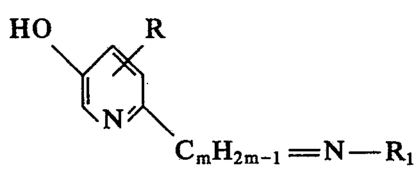

(VI)

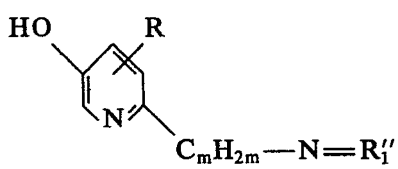

(VIa)

wherein to the carbon atom of the radical $R_1''$, which is bound to the nitrogen atom, there is bound one hydrogen atom less than is bound to the carbon atom of the radical $R_1$, is reduced; or

d) a primary amine of the general formula VII

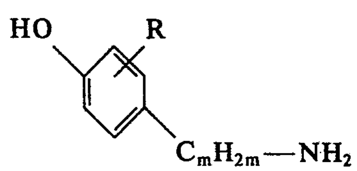

(VII)

is condensed with a reactive ester of the alcohol $R_1-OH$ in the presence of a strong base; or

e) an amide corresponding to an amine of the general formula I, wherein a carbon atom adjacent to the amino group carries an oxygen atom in place of two hydrogen atoms, is reduced; and, if desired, a compound obtained is converted into another compound of the invention, and/or, if desired, a base obtained is converted into an acid addition salt or a salt obtained is converted into the free base or into another acid addition salt, and/or, if desired, a resulting mixture of isomers or racemates is separated into the individual isomers or racemates, and/or, if desired, racemates obtained are separated into the optical antipodes.

17. The compounds obtainable by the process of claim 16.

**Claims for the Contracting State: AT**

1. Process for producing secondary 2-aminoalkyl-5-pyridinols of the general formula I

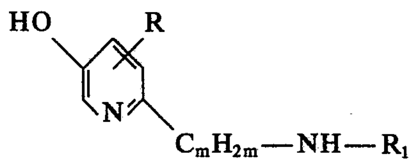

(I)

wherein R is hydrogen or alkyl having 1—4 carbon atoms, m is an integer from 2 to 4, and $R_1$ is alkyl, cycloalkyl or alkenyl having in each case at most 7 carbon atoms, the double bond in alkenyl radicals being in the 2- or 3-position, and acid addition salts thereof, characterised in that

a) to a compound of the general formula III

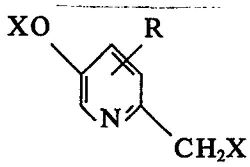

(III)

wherein X is an alkali metal atom or an alkaline-earth metal halogen group, there is added, by an addition reaction, a compound of the general formula

$$C_{m-1}H_{2m-2}=N-R_1 \qquad (IV)$$

and the product of the general formula I is liberated from the metal salt obtained; or

b) in a compound of the general formula V

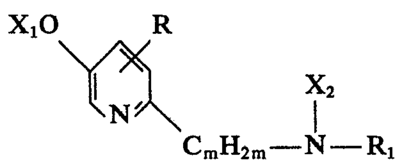

(V)

wherein each of the symbols $X_1$ and $X_2$ is hydrogen or the acyl radical of an aliphatic or aromatic carboxylic acid or sulfonic acid, with the proviso that at least one of these symbols is an acyl radical, the acyl radical $X_1$ and/or $X_2$ is replaced, by means of solvolysis or hydrogenolysis, by hydrogen; or

c) a Schiff base of the general formula VI or VIa

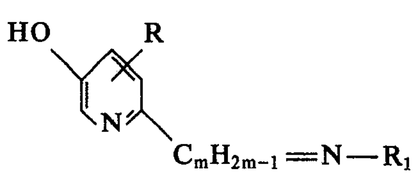

(VI)

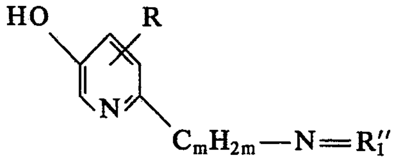

(VIa)

wherein tho the carbon atom of the radical $R_1''$, which is bound to the nitrogen atom, there is bound one hydrogen atom less than is bound to the carbon atom of the radical $R_1$, is reduced; or

d) a primary amine of the general formula VII

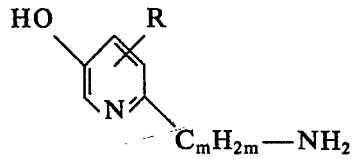

(VII)

is condensed with a reactive ester of the alcohol $R_1-OH$ in the presence of a strong base; or

e) an amide corresponding to an amine of the general formula I, wherein a carbon atom adjacent to the amino group carries an oxygen atom in place of two hydrogen atoms, is reduced; and, if desired, a compound obtained is converted into another compound of the invention, and/or, if desired, a base obtained is converted into an acid addition salt or a salt obtained is converted into the free base or into another acid addition salt, and/or, if desired, a resulting mixture of isomers or racemates is separated into the individual isomers or racemates, and/or, if desired, racemates obtained are separated into the optical antipodes.

2. Process according to claim 1, characterised in that there are used in the reaction a) starting materials of the formula III in which X is lithium, sodium or halomagnesium.

3. Process according to claim 1, characterised in that in the reaction b) the acyl radical $X_1$ and/or $X_2$ is replaced by hydrogen by treatment with agents effecting solvolysis or hydrogenolysis.

4. Process according to claim 1, characterised in that in the reaction c) the reduction is performed with catalytically activated or nascent hydrogen, or with simple or complex light metal hydrides.

5. Process according to claim 1, characterised in that in the reaction d) there are used starting materials in which the reactive ester is derived from strong inorganic acids or organic sulfonic acids.

6. Process according to claim 1, characterised in that in the reaction e) the reduction is performed with a simple or complex light metal hydride.

7. Process according to one of claims 1 to 6, characterised in that there are produced compounds of the general formula I wherein R is hydrogen or methyl, m is the integer 2 or 3, and $R_1$ is alkyl having 2 to 6 C atoms or cycloalkyl or alkenyl each having 3 to 6 C atoms, the double bond in alkenyl radicals being in the 2- or 3-position, and acid addition salts thereof.

8. Process according to one of claims 1 to 6, characterised in that there are produced compounds of the general formula II

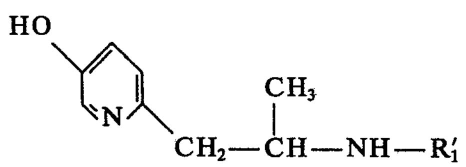

(II)

wherein $R_1'$ is alkyl, cycloalkyl or alkenyl each having 3 to 6 C atoms, the double bond in alkenyl radicals being in the 2- or 3-position, and acid addition salts thereof.

9. Process according to one of claims 1 to 6, characterised in that there are produced compounds of the formula II wherein $R_1'$ is i-propyl, tert-butyl, allyl or cyclopropyl, and acid addition salts thereof.

10. Process according to one of claims 1 to 6, characterised in that 2-(2-isopropylaminopropyl)-5-pyridinol and acid addition salts thereof are produced.

11. Process according to one of claims 1 to 10, characterised in that the resulting products are isolated in the form of their levorotatory optical antipodes.

12. Process according to claim 1 for producing 2-aminoalkyl-5-pyridinols of the general formula I given in claim 1, wherein R is hydrogen or methyl, and the other symbols have the meanings defined in claim 1, and acid addition salts thereof, characterised in that

a) to a compound of the formula II given in claim 1, wherein R is hydrogen or methyl, and X is an alkali metal atom, there is added, by an addition reaction, a compound of the formula IV given in claim 1, and the product is liberated from the metal salt obtained; or

b) a Schiff base of the formula VI given in claim 1, wherein R is hydrogen or methyl, is reduced, and, if desired, a base obtained is converted into an acid addition salt, or a salt obtained is converted into the free base or into another acid addition salt, and/or, if desired, a resulting mixture of

isomers or racemates is separated into the individual isomers or racemates, and/or, if desired, racemates obtained are separated into the optical antipodes.

13. Process according to claim 12, characterised in that in the reaction a) the liberation of the product is performed with water or with diluted inorganic or organic acids.

14. Process according to claim 12, characterised in that in the reaction b) the reduction is performed with catalytically activated or nascent hydrogen, or with simple or complex light metal hydrides.

15. Process according to one of claims 12 to 14, characterised in that there are produced compounds of the general formula I wherein R is hydrogen or methyl, m is the integer 2 or 3, $R_1$ is alkyl having 2 to 6 C atoms or cycloalkyl or alkenyl having in each case 3 to 6 C atoms, the double bond in alkenyl radicals being in the 2- or 3-position, and acid addition salts thereof.

16. Process according to one of claims 12 to 14, characterised in that there are produced compounds of the formula II given in claim 8, wherein $R_1'$ is alkyl, cycloalkyl or alkenyl each having 3 to 6 C atoms, the double bond in alkenyl radicals being in the 2- or 3-position, and acid addition salts thereof.

17. Process according to one of claims 12 to 14, characterised in that there are produced compounds of the formula II given in claim 8, wherein $R_1'$ is isopropyl or tert-butyl, and acid addition salts thereof.

18. Process according to one of claims 12 to 14, characterised in that 2-(2-isoproylaminopropyl)-5-pyridinol and acid addition salts thereof are produced.

19. Process according to one of claims 12 to 18, characterised in that the resulting products are isolated in the form of their levorotatory optical antipodes.

20. Process for producing a pharmaceutical preparation, characterised by the processing of an active ingredient according to one of claims 1 to 11 with a pharmaceutical carrier.

21. Process for producing a pharmaceutical preparation, characterised by the processing of an active ingredient according to claims 12 to 19 with a pharmaceutical carrier.

**Revendications pour les Etats contractants: BE, CH, DE, FR, IT, LI, LU, NL, SE**

1. 2-aminoalkyl-5-pyridinols secondaires de formule générale I

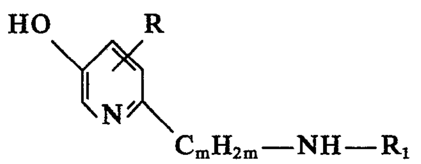

(I)

dans laquelle R représente l'hydrogène ou un groupe alkyle en C1—C4, m est un nombre entier de 2 à 4 et $R_1$ représente un groupe alkyle, cycloalkyle ou alcényle contenant chacun au maximum 7 atomes de carbone, la double liaison des restes alcényles se trouvant en position 2 ou 3, et leurs sels formés par addition avec des acides.

2. Composés selon la revendication 1, dans lesquels R représente l'hydrogène ou un groupe éthyle et les autres symboles ont les significations indiquées dans la revendication 1.

3. Composés selon la revendication 1, dans lesquels R représente l'hydrogène ou un groupe méthyle, m représente le nombre entier 2 ou 3, et $R_1$ représente un groupe alkyle en C2—C6 ou cycloalkyle ou alcényle contenant chacun 3 à 6 atomes de carbone, la double liaison des restes alcényle se trouvant en position 2 ou 3.

4. Composés de formule générale II

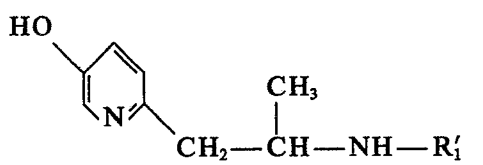

(II)

dans laquelle $R_1'$ représente un groupe alkyle, cycloalkyle ou alcényle contenant chacun 3 à 6 atomes de carbone, la double liaison des restes alcényle se trouvant en position 2 ou 3, et leurs sels formés par addition avec des acides.

5. Composés selon la revendication 4, dans lesquels $R_1'$ représente un groupe isopropyle ou tert-butyle.

6. Composés selon la revendication 4, dans lesquels $R_1'$ représente un groupe isopropyle, tert-butyle, allyle ou cyclopropyle.

7. Le 2-(2-isopropylaminopropyl)-5-pyridinol et ses sels formés par addition avec des acides.

8. Les antipodes lévogyres des composés revendiqués dans les revendications 1 et 6.

9. Les antipodes lévogyres des composés revendiqués dans les revendications 2 à 5 et 7.

10. Composés selon les revendications 1, 6 et 8 pour l'utilisation en tant qu'agents cardioprotec-

teurs.

11. Composés selon les revendications 2 à 5, 7 et 9, pour l'utilisation en tant qu'agents cardioprotecteurs.

12. Compositions pharmaceutiques contenant des composés selon l'une des revendications 1, 6, 8 et 10 ou des sels de ces composés formés par addition avec des acides utilisables en thérapeutique.

13. Compositions pharmaceutiques contenant des composés selon l'une des revendications 2 à 5, 7, 9 et 11, ou des sels de ces composés formés par addition avec des acides utilisables en thérapeutique.

14. Les composés des revendications 1, 6, 8 et 10 pour l'utilisation dans un procédé pour le traitement thérapeutique de l'organisme humain ou animal.

15. Les composés des revendications 2 à 5, 7, 9 et 11 pour l'utilisation dans un procédé pour le traitement thérapeutique de l'organisme humain ou animal.

16. Procédé de préparation des 2-aminoalkyl-5-pyridinols secondaires selon la revendication 1, caractérisé en ce que

a) sur un composé de formule générale III

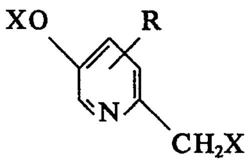

(III)

dans laquelle X représente un atome de métal alcalin ou un groupe halogéno-métal alcalino-terreux, on fixe par addition un composé de formule générale IV

$$C_{m-1}H_{2m-2}{=}N{-}R_1 \qquad \text{(IV)}$$

et on libère le produit de formule générale I à partir du sel métallique obtenu, ou bien

b) dans un composé de formule générale V

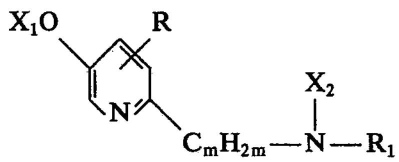

(V)

dans laquelle chacun des symboles $X_1$ et $X_2$ représente l'hydrogène ou le radical acyle d'un acide carboxylique ou sulfonique aliphatique ou aromatique, étant spécifié que l'un au moins de ces symboles représente un radical acyle, on remplace le radical acyle $X_1$ et/ou $X_2$ par l'hydrogène par solvolyse ou hydrogénolyse, ou bien

c) on réduit une base de Schiff de formule générale VI ou VIa

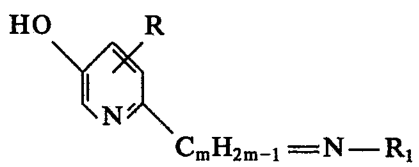

(VI)

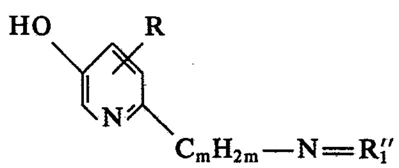

(VIa)

dans laquelle l'atome de carbone du reste $R_1''$ relié à l'atome d'azote porte un atome d'hydrogène de moins que $R_1$, ou bien

d) on condense une amine primaire de formule générale VII

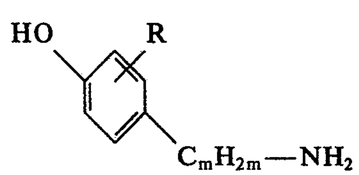

(VII)

avec un ester réactif de l'alcool $R_1-OH$ en présence d'une base forte, ou bien

e) on réduit un amide correspondant à une amine de formule générale I et dans lequel un atome de carbone voisin du groupe amino secondaire porte un atome d'oxygène à la place de deux atomes d'hydrogène et, si on le désire, on convertit un composé obtenu en un autre composé de l'invention, et/ou, si on le désire, on convertit une base obtenue en un sel formé par addition avec un acide, ou un sel obtenu en la base libre ou en un autre sel formé par addition avec un acide, et/ou, si on le désire, on sépare un mélange d'isomères ou de racémates obtenu en les isomères ou racémates individuels et/ou, si on le désire, on résout les racémates obtenus en les antipodes optiques.

17. Les composés susceptibles d'être obtenus par le procédé de la revendication 16.

**Revendications pour l'Etat Contractant: AT**

1. Procédé de préparation de 2-aminoalkyl-5-pyridinols secondaires de formule générale I

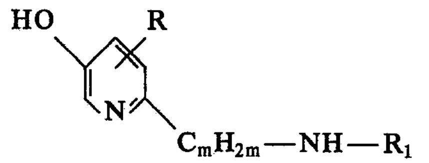

(I)

dans laquelle R représente l'hydrogène ou un groupe alkyle en C1—C4, m est un nombre entier de 2 à 4 et $R_1$ représente un groupe alkyle, cycloalkyle ou alcényle contenant chacun au maximum 7 atomes de carbone, la double liaison des restes alcényle se trouvant en position 2 ou 3, et de leurs sels formés par addition avec des acides, caractérisé en ce que

a) sur un composé de formule générale III

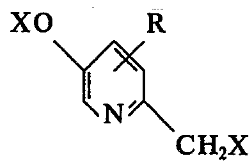

(III)

dans laquelle X représente un atome de métal alcalin ou un groupe halogéno-métal alcalino-terreux, on fixe par addition un composé de formule générale IV

$$C_{m-1}H_{2m-2}{=}N-R_1 \quad (IV)$$

et on libère le produit de formule générale I à partir du sel métallique obtenu, ou bien

b) dans un composé de formule générale V

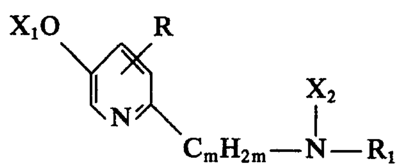

(V)

dans laquelle chacun des symboles $X_1$ et $X_2$ représente l'hydrogène ou le radical acyle d'un acide carboxylique ou sulfonique aliphatique ou aromatique, étant spécifié que l'un au moins de ces symboles représente un radical acyle, on remplace le radical acyle $X_1$ et/ou $X_2$ par l'hydrogène par solvolyse ou hydrogénolyse, ou bien

c) on réduit une base de Schiff de formule générale VI ou VIa

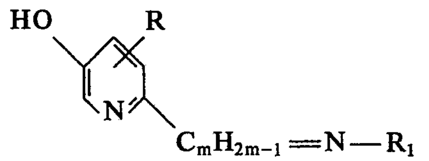

(VI)

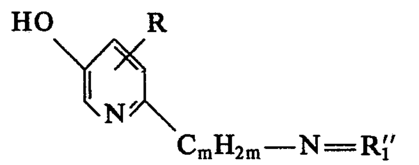

(VIa)

dans laquelle l'atome de carbone du reste $R_1''$ relié à l'atome d'azote porte un atome d'hydrogène de moins que $R_1$, ou bien

d) on condense une amine primaire de formule générale VII

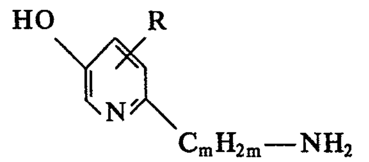

(VII)

avec un ester réactif de l'alcool $R_1-OH$ en présence d'une base forte, ou bien

e) on réduit un amide correspondant à une amine de formule générale I et dans lequel un atome de carbone voisin du groupe amino secondaire porte un atome d'oxygène à la place de deux atomes d'hydrogène et, si on le désire, on convertit un composé obtenu en un autre composé de l'invention, et/ou, si on le désire, on convertit une base obtenue en un sel formé par addition avec un acide, ou un sel obtenu en la base libre ou en un autre sel formé par addition avec un acide, et/ou, si on le désire, on sépare un mélange d'isomères ou de racémates obtenu en les isomères ou racémates individuels, et/ou, si on le désire, on résout les racémates obtenus en les antipodes optiques.

2. Procédé selon la revendication 1, caractérisé en ce que, dans la réaction a), on utilise des composés de départ de formule III dans laquelle X représente le lithium, le sodium ou le groupe halogénomagnésium.

3. Procédé selon la revendication 1, caractérisé en ce que, dans la réaction b), on remplace le radical acyle $X_1$ et/ou $X_2$ par l'hydrogène en traitant par des agents solvolysants ou hydrogénolysants.

4. Procédé selon la revendication 1, caractérisé en ce que, dans la réaction c), on effectue la réduction à l'aide d'hydrogène activé par un catalyseur ou naissant, ou à l'aide d'hydrures de métaux légers simples ou complexes.

5. Procédé selon la revendication 1, caractérisé en ce que, dans la réaction d), on utilise des produits de départ dans lesquels l'ester réactif dérive d'acides minéraux forts ou d'acides organiques sulfoniques.

6. Procédé selon la revendication 1, caractérisé en ce que, dans la réaction e), on effectue la réduction à l'aide d'un hydrure de métal léger simple ou complexe.

7. Procédé selon l'une des revendications 1 à 6, caractérisé en ce que l'on prépare des composés de formule générale I dans laquelle R représente l'hydrogène ou un groupe méthyle, m est égal à 2 ou 3, et $R_1$ représente un groupe alkyle en C2—C6 ou cycloalkyle ou alcényle contenant chacun 3 à 6 atomes de carbone, la double liaison des restes alcényle se trouvant en position 2 ou 3, et leurs sels formés par addition avec des acides.

8. Procédé selon l'une des revendications 1 à 6, caractérisé en ce que l'on prépare des composés de formule générale II

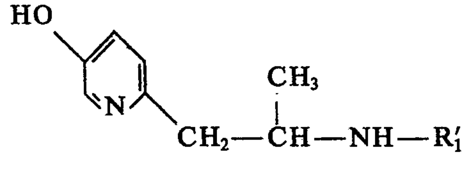

(II)

dans laquelle $R_1'$ représente un groupe alkyle, cycloalkyle ou alcényle contenant chacun 3 à 6 atomes de carbone, la double liaison des restes alcényle se trouvant en position 2 ou 3, et leurs sels formés par addition avec des acides.

9. Procédé selon l'une des revendications 1 à 6, caractérisé en ce que l'on prépare des composés de formule II dans laquelle $R_1'$ représente un groupe isopropyle, tert-butyle, allyle ou cyclopropyle, et leurs sels formés par addition avec des acides.

10. Procédé selon l'une des revendications 1 à 6, caractérisé en ce que l'on prépare le 2-(2-isopropylaminopropyl)-5-pyridinol et ses sels formés par addition avec des acides.

11. Procédé selon l'une des revendications 1 à 10, caractérisé en ce que l'on isole les produits obtenus à l'état d'antipodes lévogyres.

12. Procédé selon la revendication 1, pour la préparation des 2-aminoalkyl-5-pyridinols de formule générale I selon la revendications 1 dans laquelle R représente l'hydrogène ou le groupe méthyle et les autres symboles ont les significations indiquées dans la revendications 1, et de leurs sels formés par addition avec des acides, caractérisé en ce que

a) sur un composé de formule III de la revendication 1, dans laquelle R représente l'hydrogène ou le groupe méthyle et X un atome de métal alcalin, on fixe par addition un composé de formule IV de la revendication 1 et on libère le produit recherché à partir du sel métallique obtenu, ou bien
b) on réduit une base de Schiff de formule VI de la revendication 1 dans laquelle R représente l'hydrogène ou le groupe méthyle, et si on le désire, on convertit une base obtenue en un sel formé par addition avec un acide ou un sel obtenu en la base libre ou en un autre sel formé par addition avec un acide, et/ou, si on le désire, on sépare un mélange d'isomères ou de racémates obtenu en les isomères ou racémates individuels et/ou, si on le désire on résout les racémates obtenus en les antipodes optiques.

13. Procédé selon la revendication 12, caractérisé en ce que dans la réaction a), on libère le produit recherché à l'aide d'eau ou d'acides minéraux ou organiques dilués.

14. Procédé selon la revendication 12, caractérisé en ce que, dans la réaction b), on effectue la réduction à l'aide d'hydrogène activé par un catalyseur ou naissant ou à l'aide d'hydrures de métaux légers simples ou complexes.

15. Procédé selon l'une des revendications 12 à 14, caractérisé en ce que l'on prépare des composés de formule générale I dans laquelle R représente l'hydrogène ou un groupe méthyle, m est égal à 2 ou 3, $R_1$ représente un groupe alkyle en C2—C6 ou cycloalkyle ou alcényle contenant chacun 3 à 6 atomes de carbone, la double liaison des restes alcényle se trouvant en position 2 ou 3, et leurs sels formés par addition avec des acides.

16. Procédé selon les revendications 12 à 14, caractérisé en ce que l'on prépare des composés de formule II de la revendication 8 dans laquelle $R_1'$ représente un groupe alkyle, cycloalkyle ou alcényle contenant chacun 3 à 6 atomes de carbone, la double liaison des restes alcényle se trouvant en position 2 ou 3, et leurs sels formés par addition avec des acides.

17. Procédé selon l'une des revendications 12 à 14, caractérisé en ce que l'on prépare des composés de formule II de la revendication 8 dans laquelle $R_1'$ représente un groupe isopropyle ou tert-butyle, et leurs sels formés par addition avec des acides.

18. Procédé selon l'une des revendications 12 à 14, caractérisé en ce que l'on prépare le 2-(2-isopropylaminopropyl)-5-pyridinol et ses sels formés par addition avec des acides.

19. Procédé selon l'une des revendications 12 à 18, caractérisé en ce que l'on isole les produits obtenus à l'état d'antipodes lévogyres.

20. Procédé de préparation d'une composition pharmaceutique caractérisé en ce que l'on met en œuvre une substance active selon l'intention selon l'une des revendications 1 à 11, avec un véhicule pharmaceutique.

21. Procédé de préparation d'une composition pharmaceutique, caractérisé en ce que l'on met en œuvre une substance active selon l'invention selon l'une des revendications 12 à 19 avec un véhicule pharmaceutique.